Europäisches Patentamt

(19) European Patent Office

Office européen des brevets

(11) Publication number : **0 309 477 B1**

(12) **EUROPEAN PATENT SPECIFICATION**

(45) Date of publication of patent specification :
**06.11.91 Bulletin 91/45**

(51) Int. Cl.⁵ : **C07G 11/00, A61K 35/00, // A61K35/56**

(21) Application number : **87904158.0**

(22) Date of filing : **01.06.87**

(86) International application number :
**PCT/US87/01226**

(87) International publication number :
**WO 87/07610 17.12.87 Gazette 87/28**

(54) ECTEINASCIDINS 729, 743, 745, 759A, 759B AND 770.

(30) Priority : **09.06.86 US 872189**
**21.08.86 US 898906**
**23.01.87 US 6395**

(43) Date of publication of application :
**05.04.89 Bulletin 89/14**

(45) Publication of the grant of the patent :
**06.11.91 Bulletin 91/45**

(84) Designated Contracting States :
**AT BE CH DE FR GB IT LI LU NL SE**

(56) References cited :
**Biological Abstracts, vol. 81, 1986 (Phila.,PA, US) T. Sasaki et al.: "Antitumor activity of aqueous extracts of marine animals", see abstract no. 103819, & J. Pharmacobio-Dyn 8(11), 969-974, 1985**
**Biological Abstracts, vol. 78, 1984 (Phila.,PA, US) J.F. Davis et al.: "The enhancement of resistance of the American cel, Anguilla ros-trata, to a pathogenic bacterium, Aeromonas hydrophila, by an extract of the tunicate Ecteinascidia turbinata", see abstract no. 75713, & j. Fish Dis 7(4): 311-316, 1984**
**Chemical Abstracts, vol. 102, 1985 (Columbus, Ohio, US) M. Sigel et al.: "A substance from the marine tunicate Ecteinascidia turbinata with selective action on macrophages", see page 535, abstract no. 111299y, & Immunol. Ser. 1984, 26(Mononucl. Phagocyte Biol.), 451-71**
**Chemical Abstracts, vol. 80, 1974 (Columbus, Ohio, US) W. Lichter et al.: "Biological activi-ties exerted by extracts of Ecteinascidia tur-binata", see page 16, abstract no. 91215q & Food-Drugs Sea, Proc. (Conf.) 3rd 1972 (Pub. 1973), 117-27**

(56) References cited :
**Chemical Abstracts, vol. 84, 1976 (Columbus, Ohio, US) W. Lichter et al.: "Ecteinascidia turbinata extracts inhibit DNA synthesis in lymphocytes after mitogenic stimulation by lectins" see page 95, abstract no. 13069p, & Proc. Soc. Exp. Biol. Med. 1975, 150(2), 475-8**

(73) Proprietor : **THE BOARD OF TRUSTEES OF THE UNIVERSITY OF ILLINOIS**
**349 Administration Building, University of Illinois, 506 South Wright Street**
**Urbana, Illinois 61801 (US)**

(72) Inventor : **RINEHART, Kenneth, L.**
**1306 South Carle Avenue**
**Urbana, IL 61801 (US)**
Inventor : **HOLT, Tom, G.**
**450 Paddock Drive West**
**Savoy, IL 61874 (US)**

(74) Representative : **Ruffles, Graham Keith et al**
**MARKS & CLERK 57-60 Lincoln's Inn Fields**
**London WC2A 3LS (GB)**

EP 0 309 477 B1

## Description

DESCRIPTION

The present application relates to novel compositions of matter. More particularly, the present application relates to novel antibacterial agents designated as ecteinascidin 729, 743, 745, 759A, 759B, and 770. These compounds are extracted from the marine tunicate Ecteinascidia turbinata, which is a well-known and readily available tropical marine invertebrate. Biological activity has been assigned previously to extracts of E. turbinata; see, for example, M. -M. Sigal et al., "Anticellular and Antitumor Activity of Extracts from Tropical Marine and Vertebrates," in Food-Drugs from Sea Proceedings (1969), Youngken, H.W., Jr., Ed., Marine Technology Society,: Washington, D.C., 1970, pp 281-294; Lichter, W. et al., "Biological Activities Exerted by Extracts of Ecteinascidia turbinata," in Food-Drugs from the Sea Proceedings (1972), Worthen, L.R., Ed., Marine Technology Society: Washington, D.C., 1973, pp 117-127; Lichter, W., et al., "Inhibition of DNA Synthesis by Ecteinascidia turbinata Extracts (ETE)", in Food-Drugs from the Sea Proceedings, 1974, Webber, H.H., Ruggieri, G.D., Eds., Marine Technology Society: Washington, D.C., 1976, pp. 395-401; and Lichter, W. et al., "Immunomodulation by Extracts of Ecteinascidia turbinata", in Drugs and Food From the Sea, Kaul, P.N., Sindermann, C.J., Eds., The University of Oklahoma: Norman, OK, 1978, pp. 137-144.

INFORMATION DISCLOSURE

Extracts from Ecteinascidia turbinata are known, as described above. Certain of these extracts are known to have biological activity.

SUMMARY OF THE INVENTION

The present invention particularly provides:

(1) Ecteinascidin 729, having the following characteristics: $R_f$ = 0.28 (TLC, 5.02, 3:1 ethyl acetate-methanol), 0.26 (9:1 chloroform-methanol); HPLC retention time, 15.7 min (Whatman Partisil 10 ODS-3, 10 x 250 mm, 70:30 methanol-aqueous Tris (0.05 M), 2.8 mL/min); UV max ($CH_3OH$), 202 nm ($\varepsilon$ 61 000), 244 (sh) (11 000), 283 (5 000), 289 (4 700), (0.1 N HCl) 204 (61 000), 244 (sh) (9 600), 283 (4 800), 289 (4 500), (0.1 N KOH) 215 (33 800), 258 (8 200), 290 (6 400); IR ($CCl_4$) 3555, 3535, 2953, 2927, 2855, 1770, 1742, 1504, 1466, 1462, 1454, 1432, 1369, 1239, 1196, 1168, 1122, 1100, 1086, 1054, 1032, 997, 960 cm$^{-1}$; $^1$H NMR (360 MHzCDCl$_3$) $\delta$ 6.63 (s, 1H), 6.48 (s, 1H), 6.44 (s, 1H), 6.04 (d, $J$ = 0.7 Hz, 1H), 5.95 (d, $J$ = 0.9 Hz, 1H), 5.15 (d, $J$ = 10.7 Hz, 1H), 4.84 (bs, 1H), 4.52 (bs, 1H), 4.48 (bs, 1H), 4.38 (d, $J$ = 4.9 Hz, 1H), 4.04 (d, $J$ = 11 Hz, 1H), 3.78 (s, 3H), 3.62 (s, 3H), 3.61 (m, 2H), 3.10 (m, 1H), 3.02 (bs, 1H), 2.90 (m, 1H), 2.80 (m, 1H), 2.60 (m, 1H), 2.50 (m, 1H), 2.35 (m, 1H), 2.31 (s, 3H), 2.27 (s, 3H), 2.20 (m, 2H), 2.03 (s, 3H); FABMS, $m/z$ (rel intensity) 730(30), 495(2), 493(2), 481(2), 479(2), 463(4), 461(2), 449(4), 205(8), 204(8), 190(8); FABMS $m/z$ 730.2493; B/E linked scan on $m/z$ 729, $m/z$ 711, 696, 683, 509, 495, 481, 479, 461, 449; optical relation $[\alpha]_D^{25}$ + 112° (c 0.01, $CH_3OH$);

(2) Ecteinascidin 743, having the following characteristics: Beige solid, $R_f$ = 0.58 (3:1 ethyl acetate-methanol), 0.44 (9:1 chloroform-methanol); HPLC retention time, 18.8 minutes (Whatman Partisil 10 ODS-3, 10 x 250 mm, 70:30 methanol-aqueous Tris (0.05 M), 2.8 mL/min); UV max ($CH_3OH$) 202 nm ($\varepsilon$ 81 000), 240 (sh) (15 000), 284 (6 600), 289 (6 400), (0.1 N HCl) 205 (76 000), 240 (sh) (12 000), 285 (7 500), 289 (7 200), (0.1 N KOH) 216 (50 000), 256 (12 700), 290 (9 000). IR max ($CCl_4$) 3549, 3530, 2992 (weak), 2929, 2848, 2803 (weak), 1764, 1739, 1597 (weak), 1511, 1501, 1460, 1445, 1425, 1365, 1350, 1195, 1160, 1115, 1102, 1098, 1082, 1058, 1048, 1024, 990, 950, 915, 907 cm$^{-1}$. $^1$H NMR (500 MHz, CDCl$_3$) $\delta$ 6.62 (s, 1H), 6.48 (s, 1H), 6.46 (s, 1H), 6.03 (d, $J$ = 1.2 Hz, 1H), 5.95 (d, $J$ = 1.3 Hz, 1H), 5.71 (bs, exchanges, 1H), 5.14 (dd, $J$ = 0.9, 11.3 Hz, 1H), 4.83 (bs, 1H), 4.50 (d, $J$ = 3.3 Hz, 1H), 4.18 (d, $J$ = 4.2 Hz, 1H), 4.06 (dd, $J$ = 2.5, 11.3 Hz, 1H), 3.81 (s, 3H), 3.63 (s, 3H), 3.59 (bd, $J$ = 4.4 Hz, 1H), 3.23 (bd, $J$ = 6.5 Hz, 1H), 3.14 (ddd, $J$ = 11, 10, 4 Hz, 1H), 2.91 (bd, $J$ = 18 Hz, 1H), 2.88 (dd, $J$ = 9, 18 Hz, 1H), 2.82 (m, 1H), 2.62 (ddd, $J$ = 16, 10, 4 Hz, 1H), 2.49 (ddd, $J$ = 16, 4, 4 Hz, 1H), 2.37 (bd, $J$ = 13.9 Hz, 1H), 2.33 (s, 3H), 2.28 (s, 3H), 2.19 (s, 3H), 2.18 (d, $J$ = 13.9 Hz, 1H), 2.04 (s, 3H); $^{13}$C NMR (75.4 MHz and 125.7 MHz, CDCl$_3$) $\delta$ 9.6 (q), 15.7 (q), 20.4 (q), 24.0 (q), 28.7 (t), 39.6 (t), 41.3 (q), 42.1 (t), 42.1 (d), 54.8 (q), 55.0 (d), 55.9 (d), 57.7 (d), 57.8 (d), 60.2 (q), 61.3 (t), 64.6(s), 82.0 (d), 101.6 (t), 109.8 (d), 112.5, 114.1 (d), 115.9, 118.1(s), 120.9 (d), 121.9(s), 126.0(s), 129.2(s), 129.2(s), 131.5(s), 140.5(s), 141.3(s), 143.0(s), 144.3(s), 144.5(s), 145.1(s), 147.7(s), 168.3(s), 172.5(s); FABMS $m/z$ (rel intensity) 744.2648 (100), 699.2766 (4), 613 (10), 495.2064 (15), 477.1978 (15), 475 (9), 463.1837 (25), 218(39), 204.1027 (71). LC/FABMS $m/z$

(rel intensity) 744 (34), 495 (12), 493 (16), 477 (14), 475 (10), 463 (14), 234 (42), 218 (64), 204 (100), 189 (62), 174 (28), 160 (22); EIMS m/z 217.0737305, 191.0941620, 176.0696716. ESCA (mole percent) C(73.1), 0(20.4), N(5.2), S(1.3), optical rotation $[\alpha]_D^{25}$ + 1140 (c 0.1, $CH_3OH$);

(3) Ecteinascidin 745, having the following characteristics: $R_f$ = 0.42 (3:1 ethyl acetate-methanol, 0.38 (9:1 chloroform-methanol). HPLC retention time, 29.8 min (Whatman Partisil 10 ODS-3, 10 x 250 mm, 70:30 methanol-aqueous Tris (0.05 M), 2.8 mL/min). UV max ($CH_3OH$) 202($\varepsilon$ 52 000), 240(sh, 11 000), 281 (5 600), 287 (5 400), (0.1 N HCl), 204(51 000), 240 (sh, 9 500), 281 (5 200), 287 (5 200), (0.1 N KOH), 215 (36 000), 254 (8 500), 290 (5 900), 298 (5 800). IR ($CCl_4$ 3554, 3535, 2955, 2927, 2871, 2855, 1770, 1744, 1518, 1507, 1270, 1238, 1195, 1163, 1088, 1056 cm$^{-1}$. $^1$H NMR (360 MHz, $CDCl_3$) δ 6.62 (s, 1H), 6.52 (s, 1H), 6.47 (s, 1H), 6.02 (d, J = 1.2 Hz, 1H), 5.97 (d, J = 1,2 Hz, 1H), 5.74 (bs, exchanges, 1H), 5.14 (d, J = 11.2 Hz, 1H), 4.50 (bs, 1H), 4.29 (bt, J = 7 Hz, 1H), 4.22 (bs, 1H), 4.11 (dd, J = 11, 2 Hz, 1H), 3.80 (s, 3H), 3.68 (s, 1H), 3.63 (s, 3H), 3.31 (dd, J = 11, 2 1H), 3.23 (bs, 1H), 3.11 (m, 2H), 2.93 (m, 2H), 2.69 (m, 2H), 2.54 (m, 2H), 2.44 (d, J = 17 Hz, 1H), 2.33 (s, 3H), 2.28 (s, 3H), 2.18 (s, 3H), 2.13 (m, 1H), 2.04 (s, 3H); FABMS m/z (rel intensity) 746.2775 (100), 699 (8), 631 (8), 269 (8), 495 (19), 479 (42), 477 (52), 463 (36), 205 (64), 204 (64); LC/FABMS m/z (rel intensity) 746 (44), 495 (18), 477 (20), 463 (32), 218 (42), 204 (100), 189 (62), 176 (32), 160 (20), optical rotation $[\alpha]_D^{25}$ + 50° (c 0.1, $CH_3OH$); UV max ($CH_3OH$) 202($\varepsilon$52 000), 240(sh, 11 000), 281 (5 600), 287 (5 400), (0.1 N HCl), 204(51 000), 240 (sh, 9 500), 281 (5 200), 287 (5 200), (0.1 N KOH), 215 (36 000), 254 (8 500), 290 (5 900), 298 (5 800);

(4) Ecteinascidin 759A, having the following characteristics: LC/FABMS m/z (rel intensity) 760 (26), 509 (12), 493 (12), 463 (24), 449 (16), 246 (26), 232 (32), 224 (62), 218 (52), 204 (100), 189 (56), 174 (18), 160 (16). $R_f$ = 0.6 (3:1 ethyl acetate-methanol), 0.3 (9:1 chloroform-methanol); HPLC retention time, 11.0 min (Whatman Partisil 10 ODS-3, 10 x 250 mm, 70:30 methanol-aqueous tris (0.05 M), 2,8 mL.min); UV max ($CH_3OH$) 203 nm ($\varepsilon$ x 43 000 43), 250 (sh) (6 500), 281 (3 000), 288 (2 600), (0.1 N HCl) 205 (44 000), 250 (sh) (7 600), 281 (4 500), 288 (4 400), (0.1 N KOH) 216 (39 000), 249 (9 300), 294 (4 600); IR ($CCl_4$) 3696, 3555, 3532, 2926, 2854, 1770, 1744, 1670, 1466, 1252, 1240, 1194, 1091 cm$^{-1}$; FABMS m/z (rel intensity) 760.2563 (58), 581 (25), 493 (16), 463 (80), 461 (100) optical rotation $[\alpha]_D^{25}$ + 130° (c 0.05, $CH_3OH$);

(5) Ecteinascidin 759B, having the following characteristics: LC/FABMS m/z (rel intensity) 760 (38), 508 (8), 493 (18), 463 (26), 475 (14), 248 (30), 234 (48), 218 (86), 204 (100), 189 (56), 176 (26), 160 (32). $R_f$ = 0.6 (3:1 ethyl acetate-methanol), 0.3 (9:1 chloroform-methanol); HPLC retention time, 13.9 min (Whatman Partisil 10 ODS-3, 10 x 250 mm, 70:30 methanol-aqueous tris (0.05 M), 2.8 mL/min); UV max ($CH_3OH$) 208 nm ($\varepsilon$ x 60), 288 (4 800), 293 (4 500), (0.1 N HCl) 209 (64 000), 288 (7 100), 293 (7 100), (0.1 N KOH) 220 (45 000), 260 (10 000), 298 (7 600); IR ($CCl_4$) 3555, 2933, 1770, 1743, 1590, 1514, 1465, 1453, 1446, 1431, 1368, 1356, 1330, 1288, 1264, 1240, 1193, 1163, 1124, 1110, 1089, 1032, 1006, 821 cm$^{-1}$; FABMS m/z (rel intensity) 760.2519 (100), 744 (71), 730 (19), 493 (29), 477 (43), 463 (76); optical rotation $[\alpha]_D^{25}$ + 167° (c 0.1, $CH_3OH$); and

(6) Ecteinascidin 770, having the following charcteristics: $R_f$ = 0.6 (3:1 ethyl acetate-methanol), 0.3 (9:1 chloroform-methanol); HPLC retention time, 12.0 (Whatman Partisil 10 ODS-3, 10 x 250 mm, 70:30 methanol-aqueous tris (0.05 M), 2.8 mL/min); UV max ($CH_3OH$) 342 nm ($\varepsilon$ 3 200), 329 (3 900, 299 (22 000), 263 (25 000), 240 (58 000), 234 (55 000), 216 (66 000), (0.1 N HCl) 342 (4 900), 329 (5 700), 299 (24 000), 263 (29 000), 240 (58 000), 234 (57 000), 216 (71 000), (0.1 N KOH) 342 (3 700), 329 (4 900), 299 (22 000), 263 (28 000), 240 (58 000), 234 (57 000), 227 (57 000); IR ($CCl_4$) 3555, 3535, 3484, 2929, 2910, 1770, 1742, 1607, 1516, 1509, 1504, 1494, 1462, 1450, 1433, 1325, 1237, 1193 cm$^{-1}$; FABMS, m/z (rel intensity) 771.2682(48), 760(8), 744(20), 723(12), 613(14), 488(12), 463(14), 461(20), 205(50), 204(80).

The organism from which the ecteinascidins were extracted is a marine colonial tunicate identified as Ecteinascidia turbinata by Dr. Francoise Lafargue, Universite de Paris VI, Laboratoire Arago, Banyuls-sur-Mer, France. E. turbinata belongs to the family Perophoridae, suborder Phlebobranchia, order Enterogona, class Ascidiacea, subphylum Tunicata, phylum Chordata. Detailed descriptions of this readily available organism can be found in the following references:

1. W.G. VanName, "The Ascidians of the Bermuda Islands," Trans. Conn. Acad. Arts Sci., 11, 325-412 (1902). See pages 338-339 for a description of E. turbinata.

2. W.G. VanName, "The North and South American Ascidians," Bull. Amer. Museum Nat. Hist., 84, 1-476 (1945). See plate 20, text figures 82A, 85, 86, and pages 169-171 for a complete description of E. turbinata and a comprehensive list of previous references.

3. H.H. Plough, "Sea Squirts of the Atlantic Continental Shelf from Maine to Texas," Johns Hopkins University Press, 1978, Baltimore, MD. See text figures 13, 30e, and pages 21-22, 54, and 68 for descriptions of E. turbinata.

E. turbinata is common and widely distributed in the Caribbean. It is conspicuous on account of the large size and often bright orange color of the colonies. A colony consists of a dense cluster of elongated, club-shaped zooids, which are connected at their tapered bases by a network of stolons that adheres to the surface of the object on which the colony grows. Colonies are found in shallow water (0-20 feet) growing on mangrove roots, sponges, rocks, shells, turtle grass, bridge pilings, etc., or on bottom sand or stone. The animals are easily collected by wading, snorkeling, or SCUBA techniques.

Samples have been obtained in the following locations:

1. On islands and shores of the Indian River near the Smithsonian Tropical Research Center, Harbor Branch Foundation, Fort Pierce, Florida, 27° 27' N by 80° 20' W.

2. Between No Name Key and Big Pine Key (especially on wooden bridge pilings), Florida, 24° 42' N by 81° 21' W.

3. In the Sunshine Key Resort boat harbor of Ohio Key, Florida, 24° 40' N by 81° 14' W.

4. In the keys near St. George's Caye and Drowned Caye, Belize, 17.5° N by 88° W.

The compounds of this invention, extracted from E. turbinata. exhibit antibacterial properties and thus are useful alone or in combination with other antibacterial agents to prevent the growth of or reduce the number of susceptible bacteria in many environments.

Certain compounds of the present invention have been shown to inhibit L1210 cells and P388 leukemia cells. Thus, the compounds of this invention are useful as antitumor agents. Therefore, they are useful to inhibit the growth of tumor cells in mammals exhibiting such tumor cells. Illustratively, dosage levels of the administered active ingredients can be intravenous, 0.1 to about 200 mg/kg; intra-peritoneal, 1 to about 500 mg/kg; subcutaneous, 1 to about 500 mg/kg; intramuscular, 1 to about 500 mg/kg; oral, 5 to about 1000 mg/kg; intranasal instillation, 5 to about 1000 mg/kg; and aerosol, 5 to about 1000 mg/kg of animal (body) weight.

The compounds of the present invention are preferably presented for administration to humans and animals in unit dosage forms, such as tablets, capsules, pills, powder, granules, suppositories, syrup, sterile parenteral solutions with suspensions, sterile non-parenteral solutions with suspensions, and oral solutions or suspensions and the like containing high active quantities of the active ingredient. Those of ordinary skill in the art of pharmaceutical compositions can readily formulate compounds of the present invention into appropriate pharmaceutical compositions.

The administration of the compounds claimed herein is useful to inhibit the growth of cancer cells in animals or humans bearing a neoplastic disease, for example, acute myelocystic leukemia, acute lymphocystic leukemia, malignant melanoma, adenocarcinoma of the lung, neuroblastoma, small cell carcinoma of the lung, breast carcinoma, colon carcinoma, bladder carcinoma, and the like.

## DESCRIPTION OF THE PREFERRED EMBODIMENTS

The present is an invention as seen more fully by the examples given below.

Example 1 (Isolation and Purification)

The isolation and purification procedure was monitored at each step by performing thin-layer chromatography (TLC), in vitro antimicrobial assays, in vitro cytotoxicity assays, and bioautography on in vitro microbial organisms and tissue cell lines.

Countercurrent chromatography (CCC) employed the Ito Multi-Layer Coil Separator-Extractor with a #10 coil, a Milton Roy pump, and a LKB Uvicord II Ultraviolet (UV) detector (254, 280 nm; 3-mm flow cell). Medium pressure liquid chromatography (MPLC) was carried out with a Milton Roy pump, Ace Glass Michel-Miller Chromatography Columns, and a LKB Uvicord II UV detector (280 nm, 3-mm flow cell). Analytical TLC and bioautography were carried out on E. Merck silica gel 60 F-254 analytical TLC plates. The plates were developed in 3:1 ethyl acetate-methanol or 9:1 chloroform-methanol solvent systems and visualized with iodine vapor or a 5% solution of phosphomolybdic acid in ethanol. UV spectra were obtained in methanol on a Perkin-Elmer Lambda 3 spectrophotometer. Infrared (IR) spectra were obtained in carbon tetrachloride or chloroform on a Nicolet 7000 Fourier Transform (FT)-IR. High performance liquid chromatography (HPLC) was carried out on a Whatman Partisil 10 0DS-3 column (10 x 250 mm) and employed a Beckman 110A solvent pump and a Beckman 153 UV detector (254 nm).

Nuclear magnetic resonance (NMR) spectra were obtained on Bruker 500, General Electric 300, and Nicolet NT-360 spectrometers. Chemical shifts for $^1$H and $^{13}$C NMR spectra are reported in ppm from tetramethylsilane. Low- and high-resolution fast atom bombardment (FAB) mass spectra were obtained on VG ZAB and VG Micromass 7070 mass spectrometers. Low- and high-resolution electron ionization (EI) mass spectra were carried out on a VG ZAB mass spectrometer. Liquid chromatography/FAB mass spectrometry (LC/FABMS) employed an Alltech $C_{18}$ microbore HPLC column (10 μm, 1 x 250 nm) with a Beckman 114 solvent

4

pump, a modified VG moving belt interface for operation in the FAB mode, and a VG ZAB mass spectrometer.

Esteinascidia turbinata was collected in the Florida Keys at a depth of 0.2 to 15 feet. The sample was frozen until it was extracted by the following procedure. (Refer to Chart A. )

A sample of frozen tunicate (30.5 kg, wet weight) was thawed at room temperature and separated by coarse filtration into liquid and solid (6.3 kg) portions. A sample of the solid portion (3.8 kg) was extracted 12 times with methanol in a blender. The residue was filtered after each extraction to give a total of 20 L of methanol extract. The methanol extract was partitioned into an upper "organic" layer and a lower "polar" layer by adding 1 N aqueous $NaNO_3$ (6 L) and toluene (4 L). The aqueous phase was extracted an additional 3 times with toluene (4 L), and the toluene extracts were combined and evaporated under reduced pressure to give 26.9 g of a dark green oil. The resulting aqueous phase was extracted with dichloromethane (9 x 2L), and these extracts were combined and evaporated under reduced pressure to yield 15.9 g of a dark green solid

The dichloromethane extract was solubilized successively with dichloromethane and methanol, leaving a 5.3-g insoluble beige portion. The soluble portion was evaporated under reduced pressure to give a 10.6 g dark green solid. This material was triturated successively with hexane (20 x 20 mL) and dichloromethane (20 x 20 mL). The dichloromethane triturate (1.02 g) was purified by CCC (2 x 0.51 g) using a cyclohexane-chloroform-methanol-water solvent system (1:2:4:2) to give 8 fractions. Fractions were evaluated by TLC bioautography.

An early bioactive CCC fraction (300 mg) was chromatographed on a CHP-20 porous polymer MPLC column (18 x 350 mm) using a methanol-aqueous tris(hydroxymethyl)aminomethane (tris) (0.05 M) step gradient (85:15, 90:10, 95:5, 100:0). The chromatography was followed by UV detection (280 nm). Fractions were collected and tris was removed using a $C_{18}$ Sep-Pak, eluting first with water (to remove tris), then with methanol (to recover sample). The major bioactive component was purified further by HPLC (Whatman Partisil 10 ODS-3, 10 x 250 mm) using a 70:30 methanol-aqueous tris (0.05 M) solvent system to yield, after tris removal, ecteinascidin 743 (27 mg) and ecteinascidin 745 (4.3 mg).

A later bioactive CCC fraction (69 mg) was chromatographed on a CHP-20 porous polymer MPLC column (18 x 350 mm) using a methanol-aqueous tris (0.05 M) step gradient (80:20, 85:15, 90:10, 100:0). The chromatography was followed by UV detection (280 nm). Tris was removed from the fractions as above. The major bioactive component was purified further by HPLC (Whatman Partisil 10 ODS-3, 10 x 250 mm) using a 70:30 methanol-aqueous tris (0.05 M) solvent system to yield, after tris removal, ecteinascidin 729 (2.5 mg).

## Example 2

E. turbinata was extracted in a procedure similar to Example 1. A sample of the dichloromethane triturate (2.94 g) of the dichloromethane extract was purified by CCC (6 x 0.49 g) using a cyclohexane-chloroform-methanol-water solvent system (1:2:4:2) to give 10 fractions. Fractions were evaluated by TLC bioautography.

An early bioactive CCC fraction (200 mg) was further purified by CCC using a hexane-ethyl acetate-methanol-water (1:1:1:1) solvent system. A bioactive fraction (30 mg) was chromatographed on a CHP-20 porous polymer MPLC column (18 x 350 mm) using a methanol-water solvent system (85:15) buffered to pH 8.8 with 0.1% triethylamineacetic acid. The chromatography was followed by UV detection (280 nm). The major bioactive peak was collected (15.3 mg) and analyzed by LC/FABMS. LC/FABMS indicated one major component, ecteinascidin 743, and 3 minor components, ecteinascidin 745, ecteinascidin 759A, and ecteinascidin 759B.

## Physical Characterization of the Ecteinascidins

Ecteinascidin 729 had: $R_f = 0.28$ (TLC, 5.02, 3:1 ethyl acetate-methanol), 0.26 (9:1 chloroform-methanol); HPLC retention time, 15.7 min (Whatman Partisil 10 ODS-3, 10 x 250 mm, 70:30 methanol-aqueous Tris (0.05 M), 2.8 mL/min); UV max ($CH_3OH$), 202 nm ($\varepsilon$ 61 000), 244 (sh) (11 000), 283 (5 000), 289 (4 700), (0.1 N HCl) 204 (61 000), 244 (sh) (9 600), 283 (4 800), 289 (4 500), (0.1 N KOH) 215 (33 800), 258 (8 200), 290 (6 400); IR ($CCl_4$) 3555, 3535, 2953, 2927, 2855, 1770, 1742, 1504, 1466, 1462, 1454, 1432, 1369, 1239, 1196, 1168, 1122, 1100, 1086, 1054, 1032, 997, 960 cm$^{-1}$; $^1$H NMR (360 MHzCDCl$_3$) $\delta$ 6.63 (s, 1H), 6.48 (s, 1H), 6.44 (s, 1H), 6.04 (d, $\underline{J}$ = 0.7 Hz, 1H), 5.95 (d, $\underline{J}$ = 0.9 Hz, 1H), 5.15 (d, $\underline{J}$ = 10.7 Hz, 1H), 4.84 (bs, 1H), 4.52 (bs, 1H), 4.48 (bs, 1H), 4.38 (d, J = 4.9 Hz, 1H), 4.04 (d, $\underline{J}$ = 11 Hz, 1H), 3.78 (s, 3H), 3.62 (s, 3H), 3.61 (m, 2H), 3.10 (m, 1H), 3.02 (bs, 1H), 2.90 (m, 1H), 2.80 (m, 1H), 2.60 (m, 1H), 2.50 (m, 1H), 2.35 (m, 1H), 2.31 (s, 3H), 2.27 (s, 3H), 2.20 (m, 2H), 2.03 (s, 3H); FABMS, $\underline{m/z}$ (rel intensity) 730(30), 495(2), 493(2), 481(2), 479(2), 463(4), 461(2), 449(4), 205(8), 204(8), 190(8); FABMS $\underline{m/z}$ 730.2493; B/E linked scan on $\underline{m/z}$ 729, $\underline{m/z}$ 711, 696, 683, 509, 495, 481, 479, 461, 449; optical relation $[\alpha]_D^{25}$ + 112° ($\underline{c}$ 0.01, $CH_3OH$);

Ecteinascidin 743 a beige solid, had: Rf = 0.58 (3:1 ethyl acetate-methanol), 0.44 (9:1 chloroform-methanol); HPLC retention time, 18.8 minutes (Whatman Partisil 10 ODS-3, 10 x 250 mm, 70:30 methanol-aqueous Tris (0.05 M), 2.8 mL/min); UV max (CH$_3$OH) 202 nm ($\varepsilon$81 000), 240 (sh) (15 000), 284 (6 600), 289 (6 400), (0.1 N HI) 205 (76 000), 240 (sh) (12 000), 285 (7 500), 289 (7 200), (0.1 N KOH) 216 (50 000), 256 (12 700), 290 (9 000). IR max (CCl$_4$) 3549, 3530, 2992 (weak), 2929, 2848, 2803 (weak), 1764, 1739, 1597 (weak), 1511, 1501, 1460, 1445, 1425, 1365, 1350, 1195, 1160, 1115, 1102, 1098, 1082, 1058, 1048, 1024, 990, 950, 915, 907 cm$^{-1}$. $^1$H NMR (500 MHz, CDCl$_3$) $\delta$ 6.62 (s, 1H), 6.48 (s, 1H), 6.46 (s, 1H), 6.03 (d, $\underline{J}$ = 1.2 Hz, 1H), 5.95 (d, J = 1.3 Hz, 1H), 5.71 (bs, exchanges, 1H), 5.14 (dd, $\underline{J}$ = 0.9, 11.3 Hz, 1H), 4.83 (bs, 1H), 4.50 (d, $\underline{J}$ = 3.3 Hz, 1H), 4.18 (d, $\underline{J}$ = 4.2 Hz, 1H), 4.06 (dd, $\underline{J}$ - 2.5, 11.3 Hz, 1H), 3.81 (s, 3H), 3.63 (s, 3H), 3.59 (bd, $\underline{J}$ = 4.4 Hz, 1H), 3.23 (bd, $\underline{J}$ = 6.5 Hz, 1H), 3.14 (ddd, $\underline{J}$ = 11, 10, 4 Hz, 1H), 2.91 (bd, $\underline{J}$ = 18 Hz, 1H), 2.88 (dd, $\underline{J}$ = 9, 18 Hz, 1H), 2.82 (m, 1H), 2.62 (ddd, $\underline{J}$ = 16, 10, 4 Hz, 1H), 2.49 (ddd, $\underline{J}$ = 16, 4, 4 Hz, 1H), 2.37 (bd, $\underline{J}$ = 13.9 Hz, 1H), 2.33 (s, 3H), 2.28 (s, 3H), 2.19 (s, 3H), 2.18 (d, $\underline{J}$ = 13.9 Hz, 1H), 2.04 (s, 3H); $^{13}$C NMR (75.4 MHz and 125.7 MHz, CDCl$_3$) $\delta$ 9.6 (q), 15.7 (q), 20.4 (q), 24.0 (q), 28.7 (t), 39.6 (t), 41.3 (q), 42.1 (t), 42.1 (d), 54.8 (q), 55.0 (d), 55.9 (d), 57.7 (d), 57.8 (d), 60.2 (q), 61.3 (t), 64.6(s), 82.0 (d), 101.6 (t), 109.8 (d), 112.5, 114.1 (d), 115.9, 118.1(s), 120.9 (d), 121.9(s), 126.0(s), 129.2(s), 129.2(s), 131.5(s), 140.5(s), 141.3(s), 143.0(s), 144.3(s), 144.5(s), 145.1(s), 147.7(s), 168.3(s), 172.5(s); FABMS $\underline{m/z}$ (rel intensity) 744.2648 (100), 699.2766 (4), 613 (10), 495.2064 (15), 477.1978 (15), 475 (9), 463.1837 (25), 218(39), 204.1027 (71). LC/FABMS $\underline{m/z}$ (rel intensity) 744 (34), 495 (12), 493 (16), 477 (14), 475 (10), 463 (14), 234 (42), 218 (64), 204 (100), 189 (62), 174 (28), 160 (22); EIMS m/z 217.0737305, 191.0941620, 176.0696716. ESCA (mole percent) C(73.1), 0(20.4), N(5.2), S(1.3), optical rotation [$\alpha$]$_D^{25}$ + 1140 ($\underline{c}$ 0.1, CH$_3$OH);

Ecteinascidin 745 had: R$_f$ = 0.42 (3:1 ethyl acetate-methanol, 0.38 (9:1 chloroform-methanol). HPLC retention time, 29.8 min (Whatman Partisil 10 ODS-3, 10 x 250 mm, 70:30 methanol-aqueous Tris (0.05 M), 2.8 mL/min). UV max (CH$_3$OH) 202($\varepsilon$52 000), 240(sh, 11 000), 281 (5 600), 287 (5 400), (0.1 N HCl), 204(51 000), 240 (sh, 9 500), 281 (5 200), 287 (5 200), (0.1 N KOH), 215 (36 000), 254 (8 500), 290 (5 900), 298 (5 800). IR (CCl$_4$ 3554, 3535, 2955, 2927, 2871, 2855, 1770, 1744, 1518, 1507, 1270, 1238, 1195, 1163, 1088, 1056 cm$^{-1}$. $^1$H NMR (360 MHz, CDCl$_3$) $\delta$ 6.62 (s, 1H), 6.52 (s, 1H), 6.47 (s, 1H), 6.02 (d, $\underline{J}$ = 1.2 Hz, 1H), 5.97 (d, $\underline{J}$ = 1,2 Hz, 1H), 5.74 (bs, exchanges, 1H), 5.14 (d, $\underline{J}$ = 11.2 Hz, 1H), 4.50 (bs, 1H), 4.29 (bt, $\underline{J}$ = 7 Hz, 1H), 4.22 (bs, 1H), 4.11 (dd, $\underline{J}$ = 11, 2 Hz, 1H), 3.80 (s, 3H), 3.68 (s, 1H), 3.63 (s, 3H), 3.31 (dd, $\underline{J}$ = 11, 2 1H), 3.23 (bs, 1H), 3.11 (m, 2H), 2.93 (m, 2H), 2.69 (m, 2H), 2.54 (m, 2H), 2.44 (d, $\underline{J}$ = 17 Hz, 1H), 2.33 (s, 3H), 2.28 (s, 3H), 2.18 (s, 3H), 2.13 (m, 1H), 2.04 (s, 3H); FABMS $\underline{m/z}$ (rel intensity) 746.2775 (100), 699 (8), 631 (8), 269 (8), 495 (19), 479 (42), 477 (52), 463 (36), 205 (64), 204 (64); LC/FABMS $\underline{m/z}$ (rel intensity) 746 (44), 495 (18), 477 (20), 463 (32), 218 (42), 204 (100), 189 (62), 176 (32), 160 (20), optical rotation [$\alpha$]$_D^{25}$ + 50° ($\underline{c}$ 0.1, CH$_3$OH); UV max (CH$_3$OH) 202($\varepsilon$52 000), 240(sh, 11 000), 281 (5 600), 287 (5 400), (0.1 N HCl), 204(51 000), 240 (sh, 9 500), 281 (5 200), 287 (5 200), (0.1 N KOH), 215 (36 000), 254 (8 500), 290 (5 900), 298 (5 800);

Ecteinascidin 759A had: LC/FABMS $\underline{m/z}$ (rel intensity) 760 (26), 509 (12), 493 (12), 463 (24), 449 (16), 246 (26), 232 (32), 224 (62), 218 (52), 204 (100), 189 (56), 174 (18), 160 (16). R$_f$ = 0.6 (3:1 ethyl acetate-methanol), 0.3 (9:1 chloroform-methanol); HPLC retention time, 11.0 min (Whatman Partisil 10 ODS-3, 10 x 250 mm, 70:30 methanol-aqueous tris (0.05 M), 2.8 mL.min); UV max (CH$_3$OH) 203 nm ($\varepsilon$ x 43 000 43), 250 (sh) (6 500), 281 (3 000), 288 (2 600), (0.1 N HCl) 205 (44 000), 250 (sh) (7 600), 281 (4 500), 288 (4 400), (0.1 N KOH) 216 (39 000), 249 (9 300), 294 (4 600); IR (CCl$_4$) 3696, 3555, 3532, 2926, 2854, 1770, 1744, 1670, 1466, 1252, 1240, 1194, 1091 cm$^{-1}$; FABMS $\underline{m/z}$ (rel intensity) 760.2563 (58), 581 (25), 493 (16), 463 (80), 461 (100) optical rotation [$\alpha$]$_D^{25}$ + 130° ($\underline{c}$ 0.05, CH$_3$OH);

Ecteinascidin 759B had: LC/FABMS $\underline{m/z}$ (rel intensity) 760 (38), 508 (8), 493 (18), 463 (26), 475 (14), 248 (30), 234 (48), 218 (86), 204 (100), 189 (56), 176 (26), 160 (32). R$_f$ = 0.6 (3:1 ethyl acetate-methanol), 0.3 (9:1 chloroform-methanol); HPLC retention time, 13.9 min (Whatman Partisil 10 ODS-3, 10 x 250 mm, 70:30 methanol-aqueous tris (0.05 M), 2.8 mL/min); UV max (CH$_3$OH) 208 nm ($\varepsilon$ x 60), 288 (4 800), 293 (4 500), (0.1 N HCl) 209 (64 000), 288 (7 100), 293 (7 100), (0.1 N KOH) 220 (45 000), 260 (10 000), 298 (7 600); IR (CCl$_4$) 3555, 2933, 1770, 1743, 1590, 1514, 1465, 1453, 1446, 1431, 1368, 1356, 1330, 1288, 1264, 1240, 1193, 1163, 1124, 1110, 1089, 1032, 1006, 821 cm$^{-1}$; FABMS $\underline{m/z}$ (rel intensity) 760.2519 (100), 744 (71), 730 (19), 493 (29), 477 (43), 463 (76); optical rotation [$\alpha$]$_D^{25}$ + 167° ($\underline{c}$ 0.1, CH$_3$OH).

## Example 3 Antibacterial Activity

In a disk diffusion assay against <u>Micrococcus luteus</u>, using 0.64 cm disks, the compounds of the present invention exhibited the following results:

| Compound | Mass/disk (μg) | Zone of inhibition (mm) |
|---|---|---|
| Ecteinascidin 743 | 0.2 | 17 |
| | 0.1 | 14 |
| | 0.05 | 12 |
| | 0.02 | 9 |
| | 0.01 | 7 |
| | 0.005 | trace |
| Ecteinascidin 745 | 40 | 7 |
| | 20 | trace |
| Ecteinascidin 729 | 0.250 | 22 |
| | 0.125 | 20 |
| | 0.063 | 19 |

Bioactivity vs Micrococcus luteus. (0.64 cm disks).

| Compound | Mass/disk μg) | Zone of inhibition (mm) |
|---|---|---|
| Ecteinascidin 759A | 1 | 9 |
| | 0.1 | - |
| Ecteinascidin 759B | 1 | 15 |
| | 0.1 | 9 |
| Ecteinascidin 770 | 1 | 14 |
| | 0.1 | trace |

Example 4 L1210 Tube Dilution Assay

The compounds of this invention were shown to inhibit the growth of L1210 mouse leukemia cells in vitro as shown in the following table. The L1210 tube dilution assay is described in detail in a publication by L. H. Li, et al., Cancer Research 39:4816 (1979). $ID_{50}$ and $ID_{90}$ refer to the concentration of ecteinascidin needed to inhibit cell growth by 50 and 90 percent, respectively.

| Compound | $ID_{50}$ (μg/mL) | $ID_{90}$ (μg/mL) |
|---|---|---|
| Ecteinascidin 743 | 0.0005 | 0.0017 |
| Ecteinascidin 745 | 0.088 | 0.19 |

Example 5 In Vivo Testing of Ecteinascidins Against P388 Leukemia

Compounds of this invention are also active in vivo against P388 leukemia in mice. The P388 mouse leukemia test is described in detail in a publication by G.L. Neil et al., Cancer Treatment Reports 63, 1971-1978 (1979). The results of three P388 mouse leukemia tests using different dosage schedules is shown below. P388 in vivo

| Compound | Dose (mg/kg) | T/C |
|---|---|---|
| Ecteinascidin 743 | 0.063 | toxic |
| | 0.031 | 161 |
| Ecteinascidin 745 | 0.25 | ca. 100 |

Example 6 (Isolation of Ecteinascidin 770)

E. turbinata (48 kg) is extracted in a procedure similar to Example 1. The methanol extract was partitioned into an upper "organic" layer and a lower "polar" layer by adding IN aqueous $NaNO_3$ (4 L) and toluene (4 L, then 2 L). The toluene extracts were combined and evaporated under reduced pressure to produce a dark green oil (54 g). This material was triturated successively with hexane (10 x 50 mL, 10 x 20 mL), and dichloromethane (5 x 50 mL) to give a hexane triturate (50 g) and a dichloromethane triturate (2.1 g). A portion of the dichloromethane triturate (1.2 g) was purified by CCC (3 x 400 mg) using a cyclohexane-chloroform-methanol-water (1:2:4:2) solvent systen to give 4 fractions.

An early bioactive CCC fraction (500 mg) was chromatographed on a CHP-20 porous polymer MPLC column (18 x 350 mm) using a methanol-0.05 M tris (85.15) solvent system and UV detection (280 nm). Two bioactive peaks were collected. The first bioactive peak was purified by HPLC (70:30 methanol-0.05 M tris) to yield, after removal of buffer, ecteinascidin 759A (7 mg), ecteinascidin 759B (6 mg), and ecteinascidin 743 (5 mg). The second bioactive peak was purified by HPLC (70:30 methanol-0.05 M tris) to yield, after removal of buffer, ecteinascidin 770 (12 mg). $R_f$ = 0.6 (3:1 ethyl acetate-methanol), 0.3 (9:1 chloroform-methanol); HPLC retention time, 12.0 (Whatman Partisil 10 ODS-3, 10 x 250 mm, 70:30 methanol-aqueous tris (0.05 M), 2.8 mL/min); FABMS, m/z 771.2682; $[\alpha]^{25}_D$ +52° (c 0.1, $CH_3OH$); UV max ($CH_3OH$) 342 nm ($\varepsilon$ 3 200), 329 (3 900, 299 (22 000), 263 (25 000), 240 (58 000), 234 (55 000), 216 (66 000), (0.1 N HCl) 342 (4 900), 329 (5 700), 299 (24 000), 263 (29 000), 240 (58 000), 234 (57 000), 216 (71 000), (0.1 N KOH) 342 (3 700), 329 (4 900), 299 (22 000), 263 (28 000), 240 (58 000), 234 (57 000), 227 (57 000); IR ($CCl_4$) 3555, 3535, 3484, 2929, 2910, 1770, 1742, 1607, 1516, 1509, 1504, 1494, 1462, 1450, 1433, 1325, 1237, 1193 cm$^{-1}$; FABMS, m/z (rel intensity) 771 (48), 760 (8), 744 (20), 723 (12), 613 (14), 488 (12), 463 (14), 461 (20), 205 (50), 204 (80).
Anal. Calcd for $C_{40}H_{43}N_4O_{10}S$: 771.2700 (M + H).
Found: 771.2682.

Example 7 Chemical Degradations of Ecteinascidin 743

Part A. Treatment with KOH
Ecteinascidin 743 (1.0 mg, 1.3 μmol) is dissolved in methanolic KOH (0.2 N) and stirred at room temperature. The reaction is followed by HPLC (Whatman Partisil 5 ODS-3, 4.6 X 250 mm, 70:30 : MeOH:0.05 M Tris, 1.5 mL/min. The product peak is observed immediately (3 min) by HPLC. After the reaction is complete (4.5 min), the reaction mixture is neutralized with methanolic HCl (0.5 M) and the solvent is removed under a stream of nitrogen. The product is taken up in methanol, filtered, and purified by HPLC to yield deacetylecteinascidin 743 (0.8 mg, 85%): Pale yellow solid; $t_R$ = 7.0 min (Whatman Partisil 5 ODS-3, 4.6 x 250 mm, 70:30 : MeOH:0.05 M Tris, 1.5 mL/min); UV max ($CH_3OH$) 289 nm ($\varepsilon$ 2 000), 283 (2 000), 203 (56 000), (0.1 N KOH) 300 (5 000), 295 (5 000), 255 (8 000) 215 (30 000); IR ($CCl_4$) 3536, 2951, 2926, 2878, 2855, 1743, 1270, 1239 cm$^{-1}$; $^1$H NMR ($CDCl_3$) $\delta$ 6.63 (s,1) 6.49 (s,1), 6.41 (s,1), 5.94 (d,l,$J$ = 1 Hz), 5.87, (d,l,$J$ = 1 Hz), 5.09, (d,l,$J$ = 10.7) 4.83 (s,1), 4.50 (m,1); 4.41 (s,1), 4.19 (d,l,$J$ = 4.7 Hz) 4.01 (m,1), 3.82 (s,3), 3.65 (m,2), 3.59 (s,3), 3.23 (d,l,$J$ = 6 Hz), 3.15 (m,1), 2.9 (1), 2.8 (1), 2.5 (1), 2.4 (1), 2.35 (s,3), 2.18 (s,3), 2.15 (s,3); FABMS, m/z (rel intensity) 702 (8), 218 (4), 204 (8); B/E linked scan FABMS m/z 702, m/z 702 → m/z (rel intensity) 701 (100), 688 (4), 672 (6), 654 (24), 453 (44), 451 (28), 433 (48) 421 (20).
Anal. Calcd for: $C_{37}H_{40}N_3O_9S$: 702.2485 (M + H).
Found: 702.2438 (HRFABMS).
Part B. Treatment with Lithium Aluminum Hydride
Ecteinascidin 743 (1.0 mg, 1.3 mol) is dissolved in cold diethyl ether (0.5 mL, 0 °c). Lithium aluminum hydride (ca. 0.5 mg, 13 μmol) is added, causing the immediate evolution of gas. The reaction mixture is stirred for 30 min, then warmed to 25 °c and stirred for 1 day. When HPLC analysis indicates no reaction after 1 day, another portion of LAH (ca. 0.5 mg) is added and gas is again produced. Addition of a third portion of LAH (ca. 0.5 mg) caused no further evolution of gas. HPLC analysis at this point is no longer practical due to excessive flocculant material in the reaction mixture. The mixture is stirred another 2 days, then quenched by slow addition of methanol (0.25 mL). Following removal of solvents under a stream of nitrogen, diethyl ether (10 mL) is added. The ethereal extract is filtered, washed with water (2 x 5 mL), and dried under a stream of nitrogen to give deacetylecteinascidin 743 (0.1 mg, 11%): pale yellow solid; $t_R$ 7.2 min (Whatman Partisil 5 ODS-3, 4.6 x 250 mm, 70:30 : MeOH:0.05 M Tris, 1.5 mL/min); IR ($CCl_4$) 3536, 2951, 2926, 2874, 2855, 1740, 1270 cm$^{-1}$; FABMS, m/z (rel intensity) 744 (6), 702 (18), 453 (20), 451 (16), 435 (10), 433 (10), 421 (16), 419 (18), 218 (20), 204 (80).
Anal. Calcd for: $C_{37}H_{40}N_3O_9S$: 702.2485 (M + H).
Found: 702.2438 (HRFABMS).

Part C. Treatment with Ozone

Ecteinascidin 743 (2.3 mg, 3.1 μmol), in methanol (10 mL) is cooled to -78 °C. Ozone (Welsbach model T-816 ozonator) is bubbled through the solution until it turns light blue (3 min). Dimethyl sulfide (20 μL, 250 μmol), is added and the solution is stirred, slowly warmed ( 1 h) to room temperature, and stirred for 1 h. The product is dried under a stream of nitrogen, taken up in water (5 mL) and chloroform (5 mL), and the aqueous layer is extracted (3 x 5 mL) with chloroform to yield, after removal of solvent, an aqueous-soluble portion (3.1 mg) and an organic-soluble portion (<0.5 mg). HPLC analysis of the aqueous-soluble portion revealed the presence of a complex mixture. Attempts to isolate and purify the components of this mixture were unsuccessful.

Example 8 Derivatives of Exteinascidin 743

Part A. Preparation of Mono- and Di-O-methylecteinascidins 743

A freshly prepared ethereal solution of diazomethane [from Diazald (Aldrich), 0.25 mL] is added to a solution of ecteinascidin 743 (1 mg, 1.3 μmol) in methanol (0.25 mL). The reaction is followed by HPLC (Whatman Partisil 5 ODS-3, 4.6 x 250 mm, 70:30 : MeOH:0.05 M Tris 1.5 mL/min). An initial product ($t_R$ = 11.2 min) is observed immediately (3 min). After 70 min a second product ($t_R$ = 18.0 min) is observed in a ca. 1:1 ratio with the initial product. The reaction is stopped after 130 min by removing the solvents under a stream of nitrogen. Preparative HPLC (Whatman Partisil 10 ODS-3, 10 x 250 mm, 70:30 : MeOH:0.05 M Tris, 2.8 mL/min) of the reaction mixture afforded mono-O-methylecteinascidin 743 (0.3 mg, 29%) and di-O-methylecteinascidin 743 (0.5 mg, 48%).

Part B. Mono-O-methylecteinascidin 743

Beige solid; $t_R$ = 11.2 min (Whatman Partisil 5 ODS-3, 4.6 x 250 mm, 70:30 : MeOH:0.05 M Tris, 1.5 mL/min; UV max (CH$_3$OH) 285 nm (ε 600), 201 (21 000), (0.1 N KOH) 287 (600); IR (CCl$_4$) 2859, 2855, 2851, 1770, 1741, 1520, 1264, 1226, 1196, 1126, 1089, 1053 cm$^{-1}$; $^1$H NMR (CDCl$_3$) δ 6.62 (s,1), 6.48 (s,1), 6.41 (s,1), 6.03 (s,1), 5.95 (s,1), 5.72 (s,1), 5.13 (d,l,$J$ = 11.5 Hz), 4.81 (br s,1), 4.50 (m,2), 4.16 (d,l,$J$ = 11.5 Hz), 4.81 (br s,1), 4.50 (m,2), 4.16 (d,l,$J$ = 4.5 Hz), 4.05 (dd,l,$J$ = 3,12 Hz), 3.80 (s,3) 3.76 (s,3), 3.60 (s,3), 3.20 (1), 2.88 (1), 2.85 (1), 2.63 (1), 2.50 (1), 2.33 (s,3), 2.27 (s,3), 2.18 (s,3), 2.03 (s,3); FABMS, m/z (rel intensity) 758 (10), 477 (22), 470 (28), 218 (12), 204 (12); B/E linked scan FABMS m/z 758 → m/z (rel intensity) 757 (100), 744 (10), 730 (14), 724 (14), 712 (20), 495 (88), 477 (20), 475 (18), 465 (12), 463 (28).

Anal. Calcd for: C$_{40}$H$_{44}$N$_3$O$_{10}$S 758.2747 (M + H).

Found: 758.2754 (HRFABMS).

Part C. Di-O-methylecteinascidin 743

Beige solid, $t_R$ = 18.0 min (Whatman Partisil 5 ODS-3, 4.6 x 250 mm, 70:30 : MeOH:).05 M Tris, 1.5 mL/min); UV max (CH$_3$OH) 280 nm (ε 500), 205 (10 000), (0.1 N KOH) 285 (700); IR (CCl$_4$) 2963, 2954, 2931, 2854, 1769, 1742, 1519, 1261, 1225, 1198, 1090, 1055, 1001 cm$^{-1}$; $^1$H NMR (300 MHz, CDCl$_3$) 6.78 (s,1), 6.46 (s,1), 6.41 (s,1), 6.03 (s,1), 5.95 (s,1), 5.14 (d,l,$J$ = 10.1 Hz), 4.82 (br s,1), 4.50 (m,1), 4.12 (dd,l,$J$ = 2,5.5 Hz), 4.05 (dd,l,$J$ = 2,11), 3.92 (s,3), 3.83 (s,3), 3.76 (s,3), 3.66 (m,1), 3.60 (m,3), 3.23 (m,1), 2.88 (m,2), 2.48 (m,1), 2.29 (s,3), 2.24 (s,3), 2.17 (s,3), 2.03 (s,3); FABMS, m/z (rel intensity) 772 (24), 509 (4), 491 (6), 477 (10), 218 (20), 204 (6); B/E linked scan FABMS m/z 772 → m/z (rel intensity) 771 (100), 758 (4), 742 (6), 726 (18), 509 (100), 491 (30), 489 (30), 479 (20), 477 (40).

Anal. Calcd. for: C$_{41}$H$_{46}$N$_3$O$_{10}$S: 772.2904 (M + H).

Found: 772.2891

Part D. Preparation of Mono- and Dioxyecteinascidins 743

Aqueous hydrogen peroxide (30%, 0.5 μL) is added to a stirred methanolic solution (0.5 mL) of ecteinascidin 743 (1 mg, 1.4 μmol) cooled to 0°C. The reaction is followed by HPLC (Whatman Partisil 5 ODS-3, 4.6 x 250 mm, 70:30:MeOH:0.05 M Tris, 1.5 mL/min. The reaction mixture is stirred for 30 min, then warmed to 25 °C with stirring. After 1.5 h, an additional aliquot of hydrogen peroxide (30%, 5 μL) is added since no reaction has occurred. A third aliquot of hydrogen peroxide (30%, 5 μL) is added after 27 h and the reaction mixture is stirred for another 2 days. HPLC analyses revealed a complex mixture (ca. 16 peaks). Preparative HPLC purification of this mixture affords monooxyecteinascidin 743 (0.2 mg, 20%) and dioxyecteinascidin 743 (0.4 mg, 38%).

Part E. Monooxyecteinascidin 743

Beige solid; $t_R$ 7.0 min (Whatman Partisil 5 ODS-3, 4.6 x 250 mm, 70:30 : MeOH:0.05 M Tris, 1.5 mL/min); UV max (CH$_3$OH) 288 nm (ε 6 000), 281 (6 000), 235 (12 000), 201 (46 000), (0.1 N KOH) 291 (8 000), 260 (10 000), 213 (58 000); IR (CHCl$_3$) 1770, 1740 cm$^{-1}$; $^1$H NMR (300 MHz, CDCl$_3$) δ 6.69 (s,1), 6.48 (s,1), 6.47 (s,1), 6.03 (s,1), 5.95 (s,1), 5.19 (s,1), 5.10 (d,l,$J$ = 11.3 Hz), 4.80 (s,1), 4.73 (m,1), 4.54 (br s,2), 4.07 (d,l,$J$ = 10 Hz), 3.82 (s,3), 3.63 (s,3), 3.03 (s,3), 2.86 (s,3), 2.26 (s,3), 2.03 (s,3); FABMS, m/z (rel intensity) 760 (16), 744 (10), 463 (6), 204 (22); B/E linked scan FABMS, m/z 760 → m/z (rel intensity) 744 (100), 743 (100), 713 (76), 698 (30), 465 (34).

Anal. Calcd for: $C_{39}H_{42}N_3O_{11}S$: 760.2540 (M + H).

Found: 760.2563 (HRFABMS).

Part F. Dioxyecteinascidin 743

Beige Solid; $t_R$ 4.2 min (Whatman Partisil 5 ODS-3, 4.6 x 250 mm, 70:30 : MeOH:0.05 M Tris, 1.5 mL/min); UV max (CH$_3$OH) 282 nm (ε 12 000), 235 (22 000), 201 (78 000), (0.1 N KOH) 293 (14 000), 250 (20 000), 214 (63 000); IR (CHCl$_3$) 1770, 1740 cm⁻¹; ¹H NMR (300 MHz, CDCl$_3$) methyl singlets: δ 3.89, 3.64, 2.33, 2.29, 2.10, 2.05; FABMS, $m/z$ (rel intensity) 776 (4), 760 (4), 463 (42); B/E linked scan FABMS, $m/z$ 766 → $m/z$ (rel intensity) 759 (100), 758 (100), 713 (72), 463 (64), $m/z$ 760 → $m/z$ (rel intensity) 742 (100), 713 (34), 698 (28), 509 (36), 494 (45), 464 (30).

Anal. Calcd for: $C_{39}H_{42}N_3O_{12}S$: 776.2489 )M + H).

Found: 776.2523 (HRFABMS).

Part G. Preparation of Monoacetylecteinascidin 743

Ecteinascidin 743 (2 mg, 2.8 μmol) is dissolved in dry dichloromethane (0.5 mL). Acetic anhydride (10 μL, 28 μmole) and pyridine (20 μL, 56 μmol) are added and the reaction is stirred at 25 °C. The reaction is followed by HPLC (Whatman Partisil 5 ODS-3, 4.6 x 250 mm, 70:30 : MeOH:0.05 M Tris, 1.5 mL/min. After 1 day, starting material is no longer detected. The solvents are removed under a stream of nitrogen and the product is purified by HPLC to yield acetylecteinascidin 743 (2.0 mg, 91%). White solid; $t_R$ 11.5 min (Whatman Partisil 5 ODS-3, 4.6 x 250 mm, 70:30 : MeOH:0.05 M Tris, 1.5 mL/min; UV max (CH$_3$OH) 282 nm (ε 7 000), 201 (84 000), (0.1 N KOH) 288 (11 000), 215 (62 000); IR (CCl$_4$) 3536, 2957, 2932, 2857, 1771, 1743, 1507, 1457, 1430, 1368, 1320, 1270, 1196, 1168, 1110, 1089, 1054, 1032, 915 cm⁻¹; ¹H NMR (300 MHz, CDCl$_3$) δ 6.61 (d,2), 6.45 (d,1), 6.01 (d,1), 5.95 (d,1), 5.71 (s,1), 5.12 (d,1), 4.81 (s,1), 4.49 (bs, 2), 4.18 (d,1), 4.04 (d,1), 3.61 (s,3), 3.54 (s,3), 3.21 (m,1), 3.12 (m,1), 2.88 (m,2), 2.61 (m,2), 2.49 (dd,1), 245 (d,1), 2.33 (s,6), 2.28 (s,3), 2.24 (s,3), 2.18 (s,6), 2.01 (d,3); FABMS, $m/z$ (rel intensity) 786 (64), 744 (14), 495 (6), 493 (6), 477 (6), 463 (12), 461 (4), 218 (28), 204 (40); EIMS (70 eV), $m/z$ (rel intensity) 768 (2), 458 (28), 443 (20), 430 (10), 416 (16), 403 (16), 385 (18), 375 (12), 360 (10), 274 (24), 259 (12), 245 (10), 231 (20) 217 (60), 203 (52), 189 (48), 174 (74), 160 (48), 146 (26), 133 (100), 115 (24); B/E linked scan FABMS, $m/z$ 786 → $m/z$ (rel intensity) 771 (12), 755 (10), 743 (14), 739 (18), 728 (4), 697 (4), 495 (50), 493 (30), 477 (20), 475 (18), 463 (56), 461 (18).

Anal. Calcd for: $C_{41}H_{44}N_3O_{11}S$: 766.2697 (M + H).

Found: 786.2712 (HRFABMS).

Part H. Preparation of Di- and Tetraacetylecetinascidins 743

Ecteinascidin 743 (0.5 mg, 0.7 μmol) is stirred in a solution of pyridine-acetic anhydride (2:1, 2 mL) for 2 days. The solvents are removed under a stream of nitrogen and the residue is taken up in water (5 mL) and chloroform (5 mL). The organic layer is washed with water (3 x 5 mL) and dried (MgSO$_4$), and the solvent is removed to yield a mixture of di- and tetraacetyl derivatives (0.5 mg, 85%): pale yellow solid; FABMS, $m/z$ (rel intensity) 912 (10), 844 (8), 828 (30), 537 (2), 535 (2), 519 (4), 505 (8), 463 (4), 461 (4), 218 (18), 205 (42), 204 (36).

Part I. Preparation of p-Bromobenzoylecteinascidin 743

Ecteinascidin 743 (2 mg, 2.8 μmol), p-bromobenzoyl chloride (3.2 mg, 14 μmol, Aldrich), pyridine (2 μL, 25 μmol), and methylene chloride (0.5 ml) are combined and stirred at room temperature for 39 h. The reaction is followed by HPLC (Whatman Partisil 5 ODS-3, 4.6 x 250 mm, 70:30 : MeOH:0.05 M Tris, 1.5 mL/min). After 24 h additional amounts of p-bromobenzoyl chloride (ca. 3 mg) and pyridine (2 μL are added. The reaction is stopped after 39 h when there is no further change in the peak corresponding to the starting material. The product is purified by preparative HPLC (Whatman Partisil 10 ODS-3, 10 x 250 mm, 70:30 - 100:0 (step gradient) MeOH:0.05 M Tris, 2.8 mL/min) to yield p-bromobenzoylecteinascidin 743 (2.4 mg, 96%). White solid; UV max (CH$_3$OH) 280 nm (ε 8 000), 241 (31 000), 201 (90 000), (0.1 N KOH) 290 (8 000), 238 (31 000), 215 (50 000); IR (CCL$_4$) 3533, 2954, 2929, 2873, 2854, 1770, 1745, 1592, 1509, 1485, 1463, 1450, 1431, 1400, 1369, 1321, 1264, 1197, 1173, 1152, 1136, 1119, 1089, 1072, 1053, 1032 cm⁻¹; ¹H NMR (300 MHz, CDCl$_3$) 7.99 (d,2,$J$ = 8.5 Hz), 7.61 (d,2,$J$ = 8.5 Hz), 6.72 (s,1), 6.62 (s,1), 6.61 (s,1), 6.00 (s,1), 5.93 (s,1), 5.71 (br s,1), 5.14 (d,l,$J$ = 11.2 Hz), 4.82 (s,1), 4.49 (br s,2), 4.17, (d,l,$J$ = 4.7 Hz), 4.05 (dd,l,$J$ = 11.2,2.2 Hz), 3.80 (s,3), 3.59 (d,l,$J$ = 5.2 Hz), 3.53 (s,3), 3.22 (br d,l,$J$ = 6), 3.15 (dd,l,$J$ = 11, 5 Hz), 2.88 (br s,1), 2.85 (m,1), 2.82 (m,1), 2.65 (m,1), 2.52 (ddd,l,$J$ = 16,4,4 Hz), 2.38 (br d,l,$J$ = 14 Hz), 2.33 (s,3), 2.28 (s,3), 2.19 (m,1), 2.18 (s,3), 2.02 (s,3); FABMS $m/z$ (rel intensity) 928 (6), 926 (5), 880 (1), 848 (1), 744 (1), 509 (2), 495 (6), 493 (4), 477 (10), 463 (22), 218 (30), 204 (52).

Anal. Calcd for: $C_{46}H_{45}{}^{81}BrN_3O_{11}S$: 928.1938 (M + H).

Found: 928.1954 (HRFABMS).

Example 9 Substructures of Ecteinascidin 743

From the spectral data on ecteinascidin 743, its derivatives and the analogous ecteinascidins one can assign substructures of the molecule. Additionally, the NMR data and especially the $^1$H-$^{13}$C heteronuclear correlation NMR data allow correlation of the six $CH_3$ groups and the three aromatic CH groups. HRABMS studies show fragmentation of ecteinascidin 743 into 4 pieces — $SCH_3$, $C_{11}H_8NO_3$, $C_{15}H_{17}NO_5$, and $C_{12}H_{14}NO_2$. The molecular formulae of the three nitrogen-containing pieces, the very intense UV spectra, the aromatic $^{13}$C NMR absorptions, and the aromatic $^1$H NMR absorptions all suggest the presence of three trioxygenated tetrahydroisoquinoline units. The $C_{12}H_{14}NO_2$ fragment, the $m/z$ 204 ion in the FAB mass spectrum, must contain the N-$CH_3$ that is absent in ecteinascidin 729, one aromatic hydroxyl, and the aromatic methyl (only two oxygens - allowed in the formula).

Example 10 Bioactivities of the ecteinascidins

The antimicrobial activities (vs. *M. luteus*) and the cytotoxic activities (vs CV-1 cells) of the ecteinascidins and ecteinascidin 743 derivatives are shown in Tables 1 and 2. Within experimental error (the dilutions are accurate to within a factor of 2 error), the activities of ecteinascidins 729 and 743 in this one assay are approximately equivalent and represent the most potent compounds in the series. Ecteinascidins 759A, 759B, and 770 are four to eight times less potent, and ecteinascidin 745 is some 32 times less potent than ecteinascidins 729 and 743. All of the derivatives of ecteinascidin 743 that are prepared have antimicrobial and cytotoxic activities. The monoacetyl derivatives has enhanced activity over the original compound, whereas the other derivatives are two to eight times less active.

The ecteinascidins are not antiviral for Herpes simplex virus type I (HSV-I) or vesicular stomatitis virus (VSV). Crude extracts have no antimicrobial activity against *Escherichia coli*, *Pencillium atrovenetum* and *Saccharomyces cerevisiae*. Ecteinascidin 743 is negative in the biochemical induction assay (BIA), an assay for DNA interaction.

The antimicrobial spectra of ecteinascidins 743 and 745 are shown in Table 3, and the antitumor activities of ecteinascidins 729 and 743 are compared in Table 4. The strong antitumor activity of the lower homologue ecteinascidin 729, is especially noteworthy. This compound is clearly responsible for much of the antitumor activity of the crude extract. It is intriguing to find ecteinascidin 745 (ring opened ecteinascidin 743) essentially devoid of activity (antimicrobial, cytotoxic, and antitumor).

TABLE 1

Anti-*M. luteus* Activities of the Ecteinascidins

and Ecteinascidin 743 Derivatives (Zones of

Inhibition in mm)

| Compound | mass (ng)/disk (6.35 nm) | | | | | |
|---|---|---|---|---|---|---|
| | 1600 | 800 | 400 | 200 | 100 | 50 |
| Ecteinascidin 729 | 18 | 16 | 15 | 11 | 8 | 7 |
| Ecteinascidin 743 | 23 | 20 | 14 | 13 | 12 | 9 |
| Ecteinascidin 745 | tr | - | - | - | - | - |
| Ecteinascidin 759A | 10 | 8 | tr | - | - | - |
| Ecteinascidin 759B | 15 | 13 | 10 | tr | - | - |
| Ecteinascidin 770 | 15 | 12 | 9 | tr | - | - |
| Ecteinascidin 743 Derivatives | | | | | | |
| Deacetyl | 16 | 14 | 12 | 10 | 8 | tr |
| Mono-O-methyl | 14 | 14 | 11 | 8 | - | - |
| Di-O-methyl | 11 | 8 | - | - | - | - |
| Monoacetyl | 28 | 20 | 20 | 14 | 12 | 10 |
| *p*-Bromobenzoyl | 12 | 10 | 8 | - | - | - |

TABLE 2

Anti-CV-1 Activities of the Ecteinascidins and

Ecteinascidin 743 Derivatives (Zones of.

Inhibition in mm)

| Compound | mass (ng)/disk (6.35 nm) | | | | | |
|---|---|---|---|---|---|---|
| | 1600 | 800 | 400 | 200 | 100 | 50 |
| Ecteinascidin 729 | 18 | 16 | 13 | 10 | 10 | 9 |
| Ecteinascidin 743 | 28 | 23 | 23 | 20 | 16 | 14 |
| Ecteinascidin 745 | 14 | 9 | - | - | - | - |
| Ecteinascidin 759A | 16 | 16 | 10 | tr | - | - |
| Ecteinascidin 759B | 22 | 22 | 15 | 13 | 11 | tr |
| Ecteinascidin 770 | 25 | 24 | 20 | 16 | 14 | 10 |
| Ecteinascidin 743 Derivatives | | | | | | |
| Deacetyl | 16 | 15 | 11 | - | - | - |
| Mono-O-methyl | 26 | 25 | 23 | 20 | 22 | 13 |
| Di-O-methyl | 18 | 16 | 13 | 17 | 15 | 13 |
| Monoacetyl | 30 | 28 | 25 | 22 | 22 | 18 |
| p-Bromobenzoyl | 18 | 16 | 12 | 10 | - | - |

TABLE 3

Antimicrobial Spectrum of Ecteinascidins 743 and 745

(Zones of Inhibition in mm around 6.35 mm disk)

| Microorganism | Ecteinascidin 743 (10 µg/disk | Ecteinascidin 745 (5 µg/disk |
|---|---|---|
| Bacillis subtilis | 25 | 0 |
| Bacillus subtilis syn | 26 | 0 |
| Klebsiella pneumoniae | 20 | 0 |
| Sarcina lutea | 27 | tr |
| Sarcina lutea sens. | 35 | 18 |
| Escherichia coli | 11 | 0 |
| Pseudomonas aeruginosa | 0 | 0 |
| Staphylococcus aureus | 19 | 0 |
| Mycobacterium avium | 17 | 0 |
| Streptococcus pyogenes | NT | tr |
| Saccharomyces cerevisiae | 0 | 0 |
| Penicillium oxalicum | 10 | 0 |

## TABLE 4

### Antitumor Activity of Ecteinascidins 743 and 745 vs P388

| Compound | T/C (%) | Dose (mg/kg) |
|---|---|---|
| Ecteinascidin 729 | 214 | 0.0038 |
| Ecteinascidin 743 | 167 | 0.015 |
| Ecteinascidin 745 | 111 | 0.25 |

CHART A1

Isolation Scheme I

*Ecteinascidia turbinata* (30.5 Kg)

1. Collect and freeze
2. Thaw at room T
3. Coarse filter wet solids
4. Fine filter liquids

Filtrate
(bioactivity may be
recovered on HP-20 resin)

Solid (6.3 Kg)

3.8 Kg portion

Blend/extract with $CH_3OH$
12 x ca. 2 L

Filtrate

Filter cake

1. Add 6 L 1 N $NaNO_3$
2. Extract with $C_7H_8$
   4 x 4 L

$C_7H_8$ extract

Aqueous layer

Extract with $CH_2Cl_2$
9 x 2 L

$CH_2Cl_2$ extract

Aqueous layer

1. Remove solvent
2. Solubilize with $CH_2Cl_2$-$CH_3OH$

Insoluble portion
(5.25 g)

soluble portion

Triturate with $n$-$C_6H_{14}$
20 x 20 mL

$n$-$C_6H_{14}$ triturate

$n$-$C_6H_{14}$ insoluble

Triturate with $CH_2Cl_2$
20 x 20 mL

$CH_2Cl_2$ triturate

$CH_2Cl_2$ insoluble

1. CCC, (1:2:4:2) $c$-$C_6H_{12}$-$CHCl_3$-$CH_3OH$-$H_2O$
2. MPLC, CHP-20 (85:15) $CH_3OH$-0.05 M tris
3. HPLC, $C_{18}$ (70:30) $CH_3OH$-0.05 M tris

Ecteinascidins 729, 743, 745, 759A, 759B

## Isolation Scheme II

*Ecteinascidia turbinata* (48 Kg)

1. Collect and freeze
2. Thaw at room T
3. Coarse filter wet solids
4. Fine filter liquids

Filtrate
(bioactivity may be
recovered on HP-20 resin)

Solid

1. Blend/extract with $CH_3OH$
   12 x ca. 2 L
2. Concentrate 4:1

Filtrate

Filter cake

1. Add 2 L $CH_3OH$
2. Add 4 L 1 N $NaNO_3$
3. Extract with $C_7H_8$
   4 L, then 2 L

Aqueous layer

$C_7H_8$ extract (54 g)

1. Remove solvent
2. Triturate with $n$-$C_6H_{14}$
   10 x 50 mL, 10 x 20 mL

$n$-$C_6H_{14}$ triturate (50 g)

$n$-$C_6H_{14}$ insoluble (3 g)

Triturate with $CH_2Cl_2$
5 x 50 mL

$CH_2Cl_2$ triturate (2.1 g)

$CH_2Cl_2$ insoluble (0.9g)

1. CCC, (1:2:4:2) $c$-$C_6H_{12}$-$CHCl_3$-$CH_3OH$-$H_2O$
2. MPLC, CHP-20 (85:15) $CH_3OH$-0.05 M tris
3. HPLC, $C_{18}$ (70:30) $CH_3OH$-0.05 M tris

Ecteinascidins 743, 759A, 759B, 770

## Claims

1. <u>Ecteinascidin 729</u>, having the following characteristics: $R_f$ = 0.28 (TLC, 5.02, 3:1 ethyl acetate-methanol), 0.26 (9:1 chloroform-methanol); HPLC retention time, 15.7 min (Whatman Partisil 10 ODS-3, 10 x 250 mm, 70:10 methanol-aqueous Tris (0.05 M), 2.8 mL/min); UV max ($CH_3OH$), 202 nm ($\varepsilon$ 61 000), 244 (sh) (11 000), 283 (5 000), 289 (4 700), (0.1 N HCl) 204 (61 000), 244 (sh) (9 600), 283 (4 800), 289 (4 500), (0.1 N KOH) 215 (33 800), 258 (8 200), 290 (6 400); IR ($CCl_4$) 3555, 3535, 2953, 2927, 2855, 1770, 1742, 1504, 1466, 1462, 1454, 1432, 1369, 1219, 1196, 1168, 1122, 1100, 1086, 1054, 1032, 997, 960 cm⁻¹; ¹H NMR (360 MHzCDCl₃) δ 6.63 (s, 1H), 6.48 (s, 1H), 6.44 (s, 1H), 6.04 (d, $J$ = 0.7 Hz, 1H), 5.95 (d, $J$ = 0.9 Hz, 1H), 5.15 (d, $J$ = 10.7 Hz, 1H), 4.84 (bs, 1H), 4.52 (bs, 1H), 4.48 (bs, 1H), 4.38 (d, J = 4.9 Hz, 1H), 4.04 (d, $J$ = 11 Hz, 1H), 3.78 (s, 3H), 3.62 (s, 3H), 3.61 (m, 2H), 3.10 (m, 1H), 3.02 (bs, 1H), 2.90 (m, 1H), 2.80 (m, 1H), 2.60 (m, 1H), 2.50 (m, 1H), 2.35 (m, 1H), 2.31 (s, 3H), 2.27 (s, 3H), 2.20 (m, 2H), 2.03 (s, 3H); FABMS, $m/z$ (rel intensity) 730(30), 495(2), 493(2), 481(2), 479(2), 463(4), 461(2), 449(4), 205(8), 204(8), 190(8); FABMS $m/z$ 710.2493; B/E linked scan on $m/z$ 729, $m/z$ 711, 696, 683, 509, 495, 481, 479, 461, 449; optical relation [α]²⁵_D + 112° ($c$ 0.01, CH₃OH)

2. Ecteinascidin 743, having the following characteristics: R_f = 0.58 (3:1 ethyl acetate-methanol), 0.44 (9:1 chloroform-methanol); HPLC retention time, 18.8 minutes (Whatman Partisil 10 ODS-3, 10 x 250 mm, 70:30 methanol-aqueous Tris (0.05 M), 2.8 mL/min; UV max (CH₃OH) 202 nm (ε81 000), 240 (sh) (15 000), 284 (6 600), 289 (6 400), (0.1 N HCl) 205 (76 000), 240 (sh) (12 000), 285 (7 500), 289 (7 200), (0.1 N KOH) 216 (50 000), 256 (12 700), 290 (9 000). IR max (CCl₄) 3549, 3530, 2992 (weak), 2929, 28.48, 2803 (weak), 1764, 1739, 1597 (weak), 1511, 1501, 1460, 1445, 1425, 1365, 1350, 1195, 1160, 1115, 1102, 1098, 1082, 1058, 1048, 1024, 990, 950, 915, 907 cm⁻¹. ¹H NMR (500 MHz, CDCl₃) δ 6.62 (s, 1H), 6.48 (s, 1H), 6.46 (s, 1H), 6.03 (d, $J$ = 1.2 Hz, 1H), 5.95 (d, J = 3.3 Hz, 1H), 5.71 (bs, exchanges, 1H), 5.14 (dd, $J$ = 0.9, 11.3 Hz, 1H), 4.83 (bs, 1H), 4.50 (d, $J$ = 3.3 Hz, 1H), 4.18 (d, $J$ = 4.2 Hz, 1H), 4.06 (dd, $J$ = 2.5, 11.3 Hz, 1H), 3.81 (s, 3H), 3.63 (s, 3H), 3.59 (bd, $J$ = 4.4 Hz, 1H), 3.23 (bd, $J$ = 6.5 Hz, 1H), 3.14 (ddd, $J$ = 11, 10, 4 Hz, 1H), 2.91 (bd, $J$ = 18 Hz, 1H), 2.88 (dd, $J$ = 9, 18 Hz, 1H), 2.82 (m, 1H), 2.62 (ddd, $J$ = 16, 10, 4 Hz, 1H), 2.49 (ddd, $J$ = 16, 4, 4 Hz, 1H), 2.37 (bd, $J$ = 13.9 Hz, 1H), 2.33 (s, 3H), 2.28 (s, 3H), 2.19 (s, 3H), 2.18 (d, $J$ = 13.9 Hz, 1H), 2.04 (s, 3H); ¹³C NMR (75.4 MHz and 125.7 MHz, CDCl₃) δ 9.6 (q), 15.7 (q), 20.4 (q), 24.0 (q), 28.7 (t), 39.6 (t), 41.3 (q), 42.1 (t), 42.1 (d), 54.8 (q), 55.0 (d), 55.9 (d), 57.7 (d), 57.8 (d), 60.2 (q), 61.3 (t), 64.6(s), 82.0 (d), 101.6 (t), 109.8 (d), 112.5, 114.1 (d), 115.9, 118.1(s), 120.9 (d), 121.9(s), 126.0(s), 129.2(s), 129.2(s), 131.5(s), 140.5(s), 141.3(s), 143.0(s), 144.3(s), 144.5(s), 145.1(s), 147.7(s), 168.3(s), 172.5(s); FABMS $m/z$ (rel intensity) 744.2648 (100), 699.2766 (4), 613 (10), 495.2064 (15), 477.1978 (15), 475 (9), 463.1817 (25), 218(19), 204.1027 (71). LC/FABMS $m/z$ (rel intensity) 744 (34), 495 (12), 493 (16), 477 (14), 475 (10), 463 (14), 234 (42), 218 (64), 204 (100), 189 (62), 174 (28), 160 (22); EIMS $m/z$ 217.0737305, 191.0941620, 176.0696716. ESCA (mole percent) C(73.1), O(20.4), N(5.2), S(1.3), optical rotation [α]²⁵_D + 1140 ($c$ 0.1, CH₃OH) or a derivative thereof.

3. Ecteinascidin 745, having the following characteristics: R_f = 0.42 (3:1 ethyl acetate-methanol, 0.38 (9:1 chloroform-methanol). HPLC retention time, 29.8 min (Whatman Partisil 10 ODS-3, 10 x 250 mm, 70:30 methanol-aqueous Tris (0.05 M), 2.8 mL/min). UV max (CH₃OH) 202(ε52 000), 240(sh, 11 000), 281 (5 600), 287 (5 400), (0.1 N HCl), 204(51 000), 240 (sh, 9 500), 281 (5 200), 287 (5 200), (0.1 N KOH), 215 (36 000), 254 (8 500), 290 (5 900), 298 (5 800). IR (CCl₄ 3554, 3535, 2955, 2927, 2871, 2855, 1770, 1744, 1518, 1507, 1270, 1218, 1195, 1163, 1088, 1056 cm⁻¹. ¹H NMR (360 MHz, CDCl₃) δ 6.62 (s, 1H), 6.52 (s, 1H), 6.47 (s, 1H), 6.02 (d, $J$ = 1.2 Hz, 1H), 5.97 (d, $J$ = 1,2 Hz, 1H), 5.74 (bs, exchanges, 1H), 5.14 (d, $J$ = 11.2 Hz, 1H), 4.50 (bs, 1H), 4.29 (bt, $J$ = 7 Hz, 1H), 4.22 (bs, 1H), 4.11 (dd, $J$ = 11, 2 Hz, 1H), 3.80 (s, 3H), 3.68 (s, 3H), 3.63 (s, 3H), 3.31 (dd, $J$ = 11, 2 1H), 3.23 (bs, 1H), 3.11 (m, 2M), 2.93 (m, 2H), 2.69 (m, 2H), 2.54 (m, 2H), 2.44 (d, $J$ = 17 Hz, 1H), 2.33 (s, 3H), 2.28 (s, 3H), 2.18 (s, 3H), 2.13 (m, 1H), 2.04 (s, 3H); FABMS $m/z$ (rel intensity) 746.2775 (100), 699 (8), 631 (8), 269 (8), 495 (19), 479 (42), 477 (52), 463 (36), 205 (64), 204 (64); LC/FABMS $m/z$ (rel intensity) 746 (44), 495 (18), 477 (20), 463 (32), 218 (42), 204 (100), 189 (62), 176 (32), 160 (20), optical rotation [α]²⁵_D + 50° ($c$ 0.1, CH₃OH); UV max (CH₃OH) 202(ε52 000), 240(sh, 11 000), 281 (5 600), 287 (5 400), (0.1 N HCl), 204(51 000), 240 (sh, 9 500), 281 (5 200), 287 (5 200), (0.1 N KOH), 215 (16 000), 254 (8 500), 290 (5 900), 298 (5 800).

4. Ecteinascidin 759A, having the following characteristics: LC/FABMS $m/z$ (rel intensity) 760 (26), 509 (12), 493 (12), 463 (24), 449 (16), 246 (26), 232 (32), 224 (62), 218 (52), 204 (100), 189 (56), 174 (18), 160 (16). R_f = 0.6 (3:1 ethyl acetate-methanol), 0.3 (9:1 chloroform-methanol); HPLC retention time, 11.0 min (Whatman Partisil 10 ODS-3, 10 x 250 mm, 70:30 methanol-aqueous tris (0.05 M), 2.8 mL.min); UV max (CH₃OH) 203 nm (ε x 43 000 43), 250 (sh) (6 500), 281 (3 000), 288 (2 600), (0.1 N HCl) 205 (44 000), 250 (sh) (7 600), 281 (4 500), 288 (4 400), (0.1 N KOH) 216 (19 000), 249 (9 100), 294 (4 600); IR (CCl₄) 3696, 3555, 3532, 2926, 2854, 1770, 1744, 1670, 1466, 1252, 1240, 1194, 1091 cm⁻¹; FABMS $m/z$ (rel intensity) 760.2563 (58), 581 (25), 493 (16), 463 (80), 461 (100) optical rotation [α]²⁵_D + 130° ($c$ 0.05, CH₃OH).

5. Ecteinascidin 759B, having the following characteristics: LC/FABMS $m/z$ (rel intensity) 760 (38), 508 (8), 493 (18), 463 (26), 475 (14), 248 (30), 234 (48), 218 (86), 204 (100), 189 (56), 176 (26), 160 (32). R_f = 0.6 (3:1 ethyl acetate-methanol), 0.3 (9:1 chloroform-methanol); HPLC retention time, 13.9 min (Whatman Partisil 10 ODS-3, 10 x 250 mm, 70:30 methanol-aqueous tris (0.05 M), 2.8 mL/min); UV max (CH₃OH) 208 nm (ε x 60), 288 (4 800), 293 (4 500), (0.1 N HCl) 209 (64 000), 288 (7 100), 293 (7 100), (0.1 N KOH) 220 (45 000), 260 (10 000), 298 (7 600); IR (CCl₄) 3555, 2933, 1770, 1743, 1590, 1514, 1465, 1453, 1446, 1431, 1368, 1356, 1330, 1288, 1264, 1240, 1193, 1163, 1124, 1110, 1089, 1032, 1006, 821 cm$^{-1}$; FABMS m/z (rel intensity) 760.2519 (100), 744 (71), 730 (19), 493 (29), 477 (43), 463 (76); optical rotation $[\alpha]_D^{25}$ + 167° (c 0.1, CH$_3$OH).

6. Ecteinascidin 770, having the following characteristics: R$_f$ = 0.6 (3:1 ethyl acetate-methanol), 0.3 (9:1 chloroform-methanol); HPLC retention time, 12.0 (Whatman Partisil 10 ODS-3, 10 x 250 mm, 70:30 methanol-aqueous tris (0.05 M), 2.8 mL/min); FABMS m/z 771.2682; $[\alpha]^{25}$D +52° (c 0.1, CH$_3$OH); UV max (CH$_3$OH) 142 nm (ε 3 200), 329 (3 900), 299 (22 000), 263 (25 000), 240 (58 000), 234 (55 000), 216 (66 000), (0.1 N HCl) 342 (4 900), 329 (5 700), 299 (24 000), 263 (29 000), 240 (58 000), 234 (57 000), 216 (71 000), (0,1 N KOH) 342 (3 700), 329 (4 900), 299 (22 000), 263 (28 000), 240 (58 000), 234 (57 000), 227 (57 000); IR (CCl$_4$) 3555, 3535, 3484, 2929, 2910, 1770, 1742, 1607, 1516, 1509, 1504, 1494, 1462, 1450, 1433, 1325, 1237, 1193 cm$^{-1}$; FABMS m/z (rel intensity) 771 (48), 760 (8), 744 (20), 723 (12), 613 (14), 488 (12), 463 (14), 461 (20), 205 (50), 204 (80).

## Patentansprüche

1. Ecteinascidin 729, mit den folgenden Kennwerten: R$_f$ = 0,28 (TLC, 5,02, 3:1 Äthylacetat-Methanol), 0,26 (9:1 Chloroform-Methanol); HPLC Verweilzeit, 15,7 min (Whatman Partisil 10 ODS-3, 10 x 250 mm, 70:30 Methanol-wässeriges Tris (0,05 M), 2,8 ml/min; UV max (CH$_3$OH), 202 nm (ε 61 000), 244 (flach) (11 000), 283 (5 000), 289 (4 700), (0,1 N HCl) 204 (61 000), 244 (flach) (9 600), 283 (4 800), 289 (4 500), (0,1 N KOH) 215 (33 800), 258 (8 200), 290 (6 400); IR (CCl$_4$) 3555, 3535, 2953, 2927, 2855, 1770, 1742, 1504, 1466, 1462, 1454, 1432, 1369, 1239, 1196, 1168, 1122, 1100, 1086, 1054, 1032, 997, 960 cm$^{-1}$; $^1$H NMR (360 MHzCOCl$_3$) δ6,63 (s, 1H), 6,48 (s, 1H), 6,44 (s, 1H), 6,04 (d, J = 0,7 Hz, 1H), 5,95 (d, J = 0,9 Hz, 1H), 5,15 (d, J = 10,7 Hz, 1H), 4,84 (bs, 1H), 4,52 (bs, 1H), 4,48 (bs, 1H), 4,38 (d, J = 4,9 Hz, 1H), 4,04 (d, J = 11 Hz, 1H), 3,78 (s, 3H), 3,62 (s, 3H), 3,61 (m, 2H), 3,10 (m, 1H), 3,02 (bs, 1H), 2,90 (m, 1H), 2,80 (m, 1H), 2,60 (m, 1H), 2,50 (m, 1H), 2,35 (m, 1H), 2,31 (s, 3H), 2,27 (s, 3H), 2,20 (m, 2H), 2,03 (s, 3H); FABMS, m/z (rel. Intensität) 730(30), 495(2), 493(2), 481(2), 479(2), 463(4), 461(2), 449(4), 205(8), 204(8), 190(8); FABMS m/z 730,2493; B/E verbunder Scaner auf m/z 729, m/z 711, 696, 683, 509, 495, 481, 479, 461, 449; optische Drehung $[\alpha]_D^{25}$ + 112° (c 0,01, CH$_3$OH)

2. Ecteinascidin 743, mit den folgenden Kennwerten: R$_f$ = 0,58 (3:1 Äthylacetat-Methanol ), 0, 44 (9:1 Chloroform-Methanol ); HPLC Verweilzeit, 18,8 min (Whatman Partisil 10 ODS-3, 10 x 250 mm, 70:30 Methanol-wässeriges Tris (0,05 M), 2,8 ml/min); UV max (CH$_3$OH) 202 nm (ε 81 000), 240 (flach) (15 000), 284 (6 600), 289 (6 400), (0,1 N HCl) 205 (76 000), 240 (flach) (12 000) , 285 (7 500), 289 (7 200), (0,1 N KOH) 216 (50 000), 256 (12 700), 290 (9 000). IR max (CCl$_4$) 3549, 3530, 2992 (schwach), 2929, 2848, 2803 (schwach), 1764, 1739, 1597 (schwach), 1511, 1501, 1460, 1445, 1425, 1365, 1350, 1195, 1160, 1115, 1102, 1098, 1082, 1058, 1048, 1024, 990, 950, 915, 907 cm$^{-1}$. 1H NMR (500 MHz, CDCl$_3$) δ6,62 (s, 1H), 6,48 (s, 1H), 6,46 (s, 1H), 6,03 (d, J = 1,2 Hz, 1H), 5,95 (d, J = 1,3 Hz, 1H), 5,71 (bs, Austausch, 1H), 5,14 (doppelte Dublette, J = 0,9, 11,3 Hz, 1H), 4,83 (bs, 1H), 4,50 (d, J = 3,3 Hz, 1H), 4,18 (d, J = 4,2 Hz, 1H), 4,06 (doppelte Dublette, J = 2,5, 11,3 Hz, 1H), 3,81 (s, 3H), 3,63 (s, 3H), 3,59 (bd, J = 4,4 Hz, 1H), 3,23 (bd, J = 6,5 Hz, 1H), 3,14 (ddd, J = 11, 10, 4 Hz, 1H), 2,91 (bd, J = 18 Hz, 1H), 2,88 (doppelte Dublette, J = 9,18 Hz, 1H), 2,82 (m, 1H), 2,62 (ddd, J = 16, 10, 4 Hz, 1H), 2,49 (ddd, J = 16, 4, 4 Hz, 1H), 2,37 (bd, J = 13,9 Hz, 1H), 2,33 (s, 3H), 2,28 (s, 3H), 2,19 (s, 3H), 2,18 (d, J = 13,9 Hz, 1H), 2,04 (s, 3H); $^{13}$C NMR (75,4 MHz und 125,7 MHz, CDCl$_3$) δ 9,6 (c), 15,7 (q), 20,4 (q), 24,0 (q), 28,7 (t), 39,6 (t), 41,3 (q), 42,1 (t), 42,1 (d), 54,8 (q), 55,0 (d), 55,9 (d), 57,7 (d), 57,8 (d) 60,2 (q), 61,3 (t), 64,6(s), 82,0 (d), 101,6 (t), 109,8 (d), 112,5, 114,1 (d), 115,9, 118,1(s), 120,9 (d), 121,9(s), 126,0(s), 129,2(s), 129,2(s), 131,5(s), 140,5(s), 141,3(s), 143,0(s), 144,3(s), 144,5(s), 145,1(s), 147,7(s), 168,3(s), 172,5(s); FABMS m/z (rel. Intensität) 744,2648 (100), 699,2766 (4), 613 (10), 495,2064 (15), 477,1978 (15), 475 (g), 463,1837 (25), 218(39), 204,1027 (71). LC/FABMS m/z (rel. Intensität) 744 (34), 495 (12), 493 (16), 477 (14), 475 (10), 463 (14), 234 (42), 218 (64), 204 (100), 189 (62), 174 (28), 160 (22); EIMS m/z 217,0737305, 191,0941620, 176,0696716. ESCA (Mol-%) C(73,1), 0(20,4), N(5,2), S(1,3), optische Drehung $[\alpha]_D^{25}$ + 1140 (c 0,1, CH$_3$OH) oder ein Derivat hievon.

3. Ecteinascidin 745, mit den folgenden Kennwerten: R$_f$ = 0,42 (3:1 Äthylacetat-Methanol, 0,38 (9:1 Chloroform-Methanol). HPLC Verweilzeit, 29,8 min (Whatman Partisil 10 ODS-3, 10 x 250 mm, 70:30 Methanol-wässeriges Tris (0,05 M), 2,8 ml/min). UV max (CH$_3$OH) 202 (ε 52 000), 240 (flach, 11 000), 281 (5 600), 287 (5 400), (0,1 N HCl), 204(51 000), 240(flach, 9 500), 281 (5 200), 287 (5 200), (0,1 N KOH, 215 (36 000), 254 (8 500), 290 (5 900), 298 (5 800). IR (CCl$_4$ 3554, 3535, 2955, 2927, 2871, 2855, 1770, 1744, 1518, 1507, 1270, 1238, 1195, 1163, 1088, 1056 cm$^{-1}$. $^1$H NMR (360 MHz, CDCl$_3$) δ 6,62 (s, 1H), 6,52 (s,1H), 6,47 (s, 1H), 6,02 (d, J = 1,2 Hz, 1H), 5,97 (d, J = 1,2 Hz, 1H), 5,74 (bs, Austausch, 1H), 5,14 (d, J = 11,2 Hz, 1H), 4,50 (bs, 1H), 4,29 (bt, J = 7 Hz, 1H), 4,22 (bs, 1H), 4,11 (doppelte Dublette, J = 11, 2 Hz, 1H), 3,80 (s, 3H), 3,68 (s, 1H), 3,63 (s, 3H), 3,31 (doppelte Dublette, $\underline{J}$ = 11, 2 1H), 3,23 (bs, 1H), 3,11 (m, 2H), 2,93 (m, 2H), 2,69 (m, 2H), 2,54 (m, 2H), 2,44 (d, $\underline{J}$ = 17 Hz, 1H), 2,33 (s, 3H), 2,28 (s, 3H), 2,18 (s, 3H), 2,13 (m, 1H), 2,04 (s, 3H); FABMS $\underline{m/z}$ (rel. Intensität) 746,2775 (100), 699 (8), 631 (8), 269 (8), 495 (19), 479 (42), 477 (52), 463 (36), 205 (64), 204 (64); LC/FABMS $\underline{m/z}$ (rel. Intensität) 746 (44), 495 (18), 477 (20), 463 (32), 218 (42), 204 (100), 189 (62), 176 (32), 160 (20), optische Drehung $[\alpha]_D^{25}$ + 50° ($\underline{c}$ 0,1, CH$_3$OH); UV max (CH$_3$OH) 202 ($\varepsilon$ 52 000), 240 (flach, 11 000), 281 (5 600), 287 (5 400), (0,1 N HC1), 204 (51 000), 240 (flach, 9 500), 281 (5 200), 287 ( 5 200), (0,1 N KOH), 215 (36 000), 254 (8 500), 290 (5 900), 298 (5 800).

4. <u>Ecteinascidin 759A</u>, mit den folgenden Kennwerten: LC/FABMS $\underline{m/z}$ (rel.Intensität) 760 (26), 509 (12), 493 (12), 463 (24), 449 (16), 246 (26), 232 (32), 224 (62), 218 (52), 204 (100), 189 (56), 174 (18), 160 (16). R$_f$ = 0,6 (3:1 Äthylacetat-Methanol), 0,3 (9:1 Chloroform-Methanol); HPLC Verweilzeit, 11,0 min (Whatman Partisil 10 ODS-3, 10 x 250 mm, 70:30 Methanol-wässeriges Tris (0,05 M), 2,8 ml/min); UV max (CH$_3$OH) 203 nm ($\varepsilon$x 43 000 43), 250 (flach) (6 500), 281 (3 000), 288 (2 600), (01 N HC1) 205 (44 000), 250 (flach) (7 600), 281 (4 500), 288 (4 400), (0,1 N KOH) 216 (39 000), 249 (9 300), 294 (4 600); IR (CC1$_4$) 3696, 3555, 3532, 2926, 2854, 1770, 1744, 1670, 1466, 1252, 1240, 1194, 1091 cm$^{-1}$; FABMS $\underline{m/z}$ (rel. Intensität) 760,2563 (58), 581 (25), 493 (16), 463 (80), 461 (100) optische Drehung $[\alpha]_D^{25}$ + 130° ($\underline{c}$ 0,05, CH$_3$OH).

5. <u>Ecteinascidin 759B</u>, mit den folgenden Kennwerten: LC/FABMS $\underline{m/z}$ (rel. Intensität) 760 (38), 508 (8), 493 (18), 463 (26), 475 (14), 248 (30), 234 (48), 218 (86), 204 (100), 189 (56), 176 (26), 160 (32). R$_f$ = 0,6 (3:1 Äthylacetat-Methanol), 0,3 (9:1 Chloroform-Methanol); HPLC Verweilzeit, 13,9 min (Whatman Partisil 10 ODS-3, 10 x 250 mm, 70:30 Methanol-wässeriges Tris (0,5 M), 2,8 ml/min); UV max (CH$_3$OH) 208 nm ($\varepsilon$ x 60), 288 (4 800), 293 (4 500), (0,1 N HC1) 209 (64 000), 288 (7 100), 293 (7 100), (0,1 N KOH) 220 (45 000), 260 (10 000), 298 (7 600); IR (CC1$_4$) 3555, 2933, 1770, 1743, 1590, 1514, 1465, 1453, 1446, 1431, 1368, 1376, 1330, 1288, 1264, 1240, 1193, 1163, 1124, 1110, 1089, 1032, 1006, 821 cm$^{-1}$; FABMS $\underline{m/z}$ (rel.Intensität) 760,2519 (100), 744 (71), 730 (19), 493 (29), 477 (43), 463 (76); optische Drehung $[\alpha]_D^{25}$ + 167° ($\underline{c}$ 0,1, CH$_3$OH).

6. <u>Ecteinascidin 770</u>, mit den folgenden Kennwerten: R$_f$ = 0,6 (3:1 Äthylacetat-Methanol ), 0, 3 ( 9:1 Chloroform-Methanol ); HPLC Verweilzeit, 12,0 (Whatman Partisil 10 ODS-3, 10 x 250 mm, 70:30 Methanol-wässeriges Tris (0,05 M), 2, 8 ml/min; FABMS $\underline{m/z}$ 771,2682; $[\alpha]_D^{25}$ + 52° (c 0, 1, CH$_3$OH); UV max (CH$_3$OH) 342 nm ($\varepsilon$ 3 200), 329 (3 900), 299 (22 000), 263 (25 000), 240 (58 000), 234 (55 000), 216 (66 000), (0,1 N HC1) 342 (4 900), 329 (5 700), 299 (24 000), 263 (29 000), 240 (58 000), 234 (57 000), 216 (71 000), (0,1 N KOH) 342 (3 700), 329 (4 900), 299 (22 000), 263 (28 000), 240 (58 000), 234 (57 000), 227 (57 000); IR (CC1$_4$) 3555, 3535, 3484, 2929, 2910, 1770, 1742, 1607, 1516, 1509, 1504, 1494, 1462, 1450, 1433, 1325, 1237, 1193 cm$^{-1}$; FABMS $\underline{m/z}$ (rel. Intensität) 771 (48), 760 (8), 744 (20), 723 (12), 613 (14), 488 (12), 463 (14), 461 (20), 205 (50), 204 (80).

## Revendications

1. <u>Ectéinascidine 729</u>, ayant les caractéristiques suivantes: R$_f$ - 0.28 (TLC, 5.02, 3:1 acétate d'éthyle-méthanol), 0.26 (9:1 chloroforme-méthanol); temps de rétention sur HPLC, 15.7 min (Whatman Partisil 10 ODS-3, 10 x 250 mm, 70:30 méthanol-Tris aqueux (0.05 M), 2.8 mL/min); UV max (CH$_3$OH), 202 mm ($\varepsilon$ 61 000), 244 (sh) (11 000), 283 (5 000), 289 (4 700), (0.1 N HCl) 204 (61 000), 244 (sh) (9 600), 283 (4 800); 289 (4 500), (0.1 N KOH) 215 (33 800), 258 (8 200), 290 (6 400); IR (CCl$_4$ 3555, 3535, 2953, 2927, 2855, 1770, 1742, 1504, 1466, 1462, 1454, 1432, 1369, 1239, 1196, 1168, 1122, 1100, 1086, 1054, 1032, 997, 960 cm$^{-1}$; $^1$H NMR (360 MHzCDCl$_3$) δ 6.63 (s, 1H), 6.48 (s, 1H), 6.44 (s, 1H), 6.04 (d, $\underline{J}$ - 0.7 Hz, 1H), 5.95 (d, $\underline{J}$ - 0.9 Hz, 1H), 5.15 (d, $\underline{J}$ - 10.7 Hz, 1H), 4.84 (bs, 1H), 4.52 (bs, 1H), 4.48 (bs, 1H), 4.38 (d, $\underline{J}$ - 4.9 Hz 1H), 4.04 (d, $\underline{J}$ - 11 Hz, 1H), 3.78 (s, 3H), 3.62 (s, 3H), 3.61 (m, 2H), 3.10 (m, 1H), 3.02 (bs, 1H), 2.90 (m, 1H), 2.80 (m, 1H), 2.60 (m, 1H), 2.50 (m, 1H), 2.35 (m, 1H), 2.31 (s, 3H), 2.27 (s, 3H), 2.20 (m, 2H), 2.03 (s, 3H); FABMS, $\underline{m/z}$ (intensité relative) 730(30), 495(2), 493(2), 481(2), 479(2), 463(4), 461(2), 449(4), 205(8), 204(8), 190(8); FABMS $\underline{m/z}$ 730.2493; balayage couplé B/E sur $\underline{m/z}$ 729, $\underline{m/z}$ 711, 696, 683, 509, 495, 481, 479, 461, 449; rotation optique $[\alpha]_D^{25}$ + 112° ($\underline{c}$ 0.01, CH$_3$OH)

2. <u>Ectéinascidine 743</u>, ayant les caractéristiques suivantes: R$_f$ - 0.58 (3:1 acétate d'éthyle-méthanol), 0.44 (9:1 chloroforme-méthanol); temps de rétention sur HPLC, 18.8 minutes (Whatman Partisil 10 ODS-3, 10 x 250 mm, 70:30 méthanol-Tris aqueux (0.05 M), 2.8 mL/min); UV max (CH$_3$OH) 202 nm ($\varepsilon$81 000), 240 (sh) (15 000), 284 (6 600), 289 (6 400), (0.1 N HCl) 205 (76 000), 240 (sh), (12 000), 285 (7 500), 289 (7 200), (0.1 N KOH) 216 (50 000), 256 (12 700), 290 (9 000), IR max (CCl$_4$) 3549, 3530, 2992 (faible), 2929, 2848, 2803 (faible), 1764, 1739, 1597 (faible), 1511, 1501, 1460, 1445, 1425, 1365, 1350, 1195, 1160, 1115, 1102, 1098, 1082, 1058, 1048, 1024, 990, 950, 915, 907 cm$^{-1}$, $^1$H NMR (500 MHz, CDCl$_3$) δ 6.62 (s, 1H), 6.48 (s, 1H), 6.46 (s, 1H), 6.03 (d, $\underline{J}$ - 1.2 Hz, 1H), 5.95 (d, J - 1.3 Hz, 1H), 5.71 (échanges bs, 1H), 5,14 (dd, $\underline{J}$ - 0.9, 11.3 Hz, 1H), 4.83 (bs, 1H), 4.50 (d, $\underline{J}$ - 3.3 Hz, 1H), 4.18 (d, $\underline{J}$ - 4,2 Hz, 1H), 4,06 (dd, $\underline{J}$ - 2.5, 11.3 Hz, 1H), 3.81 (s, 1H), 3.63 (s, 3H), 3.59 (bd, $\underline{J}$ - 4.4 Hz, 1H), 3.23 (bd, $\underline{J}$ - 6.5 Hz, 1H), 3.14 (ddd, $\underline{J}$ - 11, 10, 4 Hz, 1H), 2.91 (bd, $\underline{J}$ - 18 Hz, 1H), 2,88 (dd, $\underline{J}$ - 9, 18 Hz), 2.82 (m, 1H), 2.62 (ddd, $\underline{J}$ - 16, 10, 4 Hz, 1H), 2.49 (ddd, $\underline{J}$ - 16, 4, 4 Hz, 1H), 2.37 (bd, $\underline{J}$ - 13.9 Hz, 1H), 2.33 (s, 3H), 2.28 (s, 3H), 2.19 (s, 3H), 2.18 (d, $\underline{J}$ - 13.9 Hz, 1H), 2.04 (s, 3H); $^{13}$C NMR (75.4 MHz et 125.7 MHz, CDCl$_3$) δ 9.6 (q), 15.7 (q), 20.4 (q), 24.0 (q), 28.7 (t), 39.6 (t), 41.3 (q), 42.1 (t), 42.1 (d), 54.8 (q), 55.0 (d), 55.9 (d), 57.7 (d), 57.8 (d), 60.2 (q), 61.3 (t), 64.6 (s), 82.0 (d), 101.6 (t), 109.8 (d), 112.5 114.1 (d), 115.9, 118.1(s), 120.9 (d), 121.9(s), 126.0(s), 129.2(s), 129.2(s), 131.5(s), 140.5(s), 141.3(s), 143.0(s), 144.3(s), 144.5(s), 145.1(s), 147.7(s), 168.3(s), 172.5(s); FABMS $\underline{m/z}$ (intensité relative) 744.2648 (100), 699.2766 (4), 613 (10), 495.2064 (15), 477.1978 (15), 475 (9), 463.1837 (25), 218(39), 204.1027 (71), LC/FABMS $\underline{m/z}$ (intensité relative) 744 (34), 495 (12), 493 (16), 477 (14), 475 (10), 463 (14), 234 (42), 218 (64), 204 (100), 189 (62) 174 (28), 160 (22), 160 (22); EIMS $\underline{m/z}$ 217.0737305, 191.0941620, 176.0696716. ESCA (pourcentage molaire) C(73.1), 0(20.4), N(5.2), S(1.3), rotation optique $[\alpha]_D^{25}$ + 1140 ($\underline{c}$ 0.1, CH$_3$OH) ou un de ses dérivés.

3. Ectéinascidine 745, ayant les caractéristiques suivantes: R$_f$ - 042 (3:1 acétate d'éthyle-méthanol, 0.38 (9:1 chloroforme-méthanol). Temps de rétention sur HPLC, 29.8 min (Whatman Partisil 10 ODS-3, 10 x 250 mm, 70:30 méthanol-Tris aqueux (0.05 M), 2.8 mL/min. UV max (CH$_3$OH) 202 (ε 52 000), 240 (sh, 11 000), 281 (5 600), 287 (5 400), (0.1 N HCl), 204(51 000), 240 (sh 9 500), 281 (5 200), 287 (5 200), (0.1 N KOH), 215 (36 000), 254 (8 500), 290 (5 900), 298 (5 800). IR (CCl$_4$ 3554, 3535, 2955, 2927, 2871, 2855, 1770, 1744, 1518, 1507, 1270, 1238, 1195, 1163, 1088, 1056 cm$^{-1}$. $^1$H NMR (360 MHz, CDCl$_3$) δ 6.62 (s, 1H), 6.52 (s, 1H), 6.47 (s, 1H), 6.02 (d, $\underline{J}$ - 1.2 Hz, 1H), 5.97 (d, $\underline{J}$ - 1.2 Hz, 1H), 5.74 (échanges, bs, 1H), 5.14 (d, $\underline{J}$ - 11.2 Hz, 1H), 4.50 (bs, 1H), 4.29 (bt, $\underline{J}$ - 7 Hz, 1H), 4.22 (bs, 1H), 4.11 (dd, $\underline{J}$ - 11, 2 Hz, 1H), 3.80 (s, 3H), 3.68 (s, 1H), 3.63 (s, 3H), 3.31 (dd, $\underline{J}$ - 11, 2 1H), 3.23 (bs, 1H), 3.11 (m, 2H), 2.93 (m, 2H), 2.69 (m, 2H), 2.54 (m, 2H), 2.44 (d, $\underline{J}$ - 17 Hz, 1H), 2.33 (s, 3H), 2.28 (s, 3H), 2.18 (s, 3H), 2.13 (m, 1H), 2.04 (s, 3H); FABMS $\underline{m/z}$ (intensité relative) 746.2775 (100), 699 (8), 631 (8), 269 (8), 495 (19), 479 (42), 477 (52), 463 (36), 205 (64), 204 (64); LC/FABMS $\underline{m/z}$ (intensité relative) 746 (44), 495 (18), 477 (20), 463 (32), 218 (42), 204 (100), 189 (62), 176 (32), 160 (20), rotation optique $[\alpha]_{D25}$ + 50° ($\underline{c}$ 0.1, CH$_3$OH); UV max (CH$_3$OH) 202(ε52 000), 240(sh, 11 000), 281 (5 600), 287 (5 400), (0.1 N HCl), 204(51 000), 240 (sh, 9 500), 281 (5 200), 287 (5 200), (0.1 N KOH), 215 (36 000), 254 (8 500), 290 (5 900), 298 (5 800).

4. Ectéinascidine 759A, ayant les caractéristiques suivantes: LC/FABMS $\underline{m/z}$ (intensité relative) 760 (26), 509 (12), 493 (12), 463 (24), 449 (16), 246 (26), 232 (32), 224 (62), 218 (52), 204 (100), 189 (56), 174 (18), 160 (16). R$_f$ - 0.6 (3:1 acétate d'éthyle-méthanol), 0.3 (9:1 chloroformes-méthanol); temps de rétention sur HPLC, 11.0 min (Whatman Partisil 10 ODS-3, 10 x 250 mm, 70:30 méthanol-Tris aqueux (0.05 M), 2.8 mL/min; UV max (CH$_3$OH) 203 nm (ε x 43 000 43), 250 (sh) (6 500), 281 (3 000), 288 (2 600), (0.1 N HCl) 205 (44 000), 250 (sh) (7 600), 281 (4 500), 288 (4 400), (0.1 N KOH) 216 (39 000), 249 (9 300), 294 (4 600); IR (CCl$_4$) 3696, 3555, 3532, 2926, 2854, 1770, 1744, 1670, 1466, 1252, 1240, 1194, 1091 cm$^{-1}$; FABMS $\underline{m/z}$ (intensité relative) 760.2563 (58), 581 (25), 493 (16), 463 (80), 461 (100) rotation optique $[\alpha]_D^{25}$ + 130° ($\underline{c}$ 0.05, CH$_3$OH).

5. Ectéinascidine 759B, ayant les caractéristiques suivantes: LC/FABMS $\underline{m/z}$ (intensité relative) 760 (38), 508 (8), 493 (18), 463 (26), 475 (14), 248 (30), 234 (48), 218 (86), 204 (100), 189 (56), 176 (26), 160 (32). R$_f$ - 0.6 (3:1 acétate d'éthyle-méthanol), 0.3 (9:1 chloroforme-méthanol); temps de rétention sur HPLC, 13.9 min (Whatman Partisil 10 ODS-3, 10 x 250 mm, 70:30 méthanol-Tris aqueux (0.05 M), 2.8 mL/min); UV max (CH$_3$OH) 208 nm (ε x 60), 288 (4 800), 293 (4 500), (0.1 N HCl) 209 (64 000), 288 (7 100), 293 (7 100), (0.1 N KOH) 220 (45 000), 260 (10 000), 298 (7 600); IR (CCl$_4$) 3555, 2933, 1770, 1743, 1590, 1514, 1465, 1453, 1446, 1431, 1368, 1356, 1330, 1288, 1264, 1240, 1193, 1163, 1124, 1110, 1089, 1032, 1006, 821 cm$^{-1}$; FABMS $\underline{m/z}$ (intensité relative) 760.2519 (100), 744 (71), 730 (19), 493 (29), 477 (43), 463 (76); rotation optique $[\alpha]_D^{25}$ + 167° ($\underline{c}$ 0.1, CH$_3$OH).

6. Ectéinascidine 770, ayant les caractéristiques suivantes: R$_f$ - 0.6 (3:1 acétate d'éthyle-méthanol), 0.3 (9:1 chloroforme-méthanol); temps de rétention sur HPLC, 12.0 min (Whatman Partisil 10 ODS-3, 10 x 250 mm, 70:30 méthanol-Tris aqueux (0.05 M), 2.8 mL/min); FABMS $\underline{m/z}$ 771.2682; $[\alpha]^{25}_D$ + 52° (c 0.1, CH$_3$OH); UV max (CH$_3$OH) 342 nm (ε 3 200), 329 (3 900), 299 (22 000), 263 (25 000); 240 (58 000), 234 (55 000), 216 (66 000), (0.1 N HCl) 342 (4 900), 329 (5 700), 299 (24 000), 263 (29 000), 240 (58 000), 234 (57 000), 216 (71 000), (0.1 N KOH) 342 (3 700), 329 (4 900), 299 (22 000), 263 (28 000), 240 (58 000), 234 (57 000), 227 (57 000); IR (CCl$_4$) 3555, 3535, 3484, 2929, 2910, 1770, 1742, 1607, 1516, 1509, 1504, 1494, 1462, 1450, 1433, 1325, 1237, 1193 cm$^{-1}$; FABMS $\underline{m/z}$ (intensité relative) 771 (48), 760 (8), 744 (20), 723 (12), 613 ( 14), 488 (12), 463 (14), 461 (20), 205 (50), 204 (80).